(19) Europäisches Patentamt
European Patent Office
Office européen des brevets

(11) EP 0 502 473 B1

(12) # EUROPÄISCHE PATENTSCHRIFT

(45) Veröffentlichungstag und Bekanntmachung des Hinweises auf die Patenterteilung:
**26.04.2000 Patentblatt 2000/17**

(51) Int. Cl.[7]: **C07D 285/14**, A01N 43/82

(21) Anmeldenummer: **92103607.5**

(22) Anmeldetag: **03.03.1992**

(54) **Mikrobizide Benzo-1,2,3-thiadiazolderivate**
Microbicidal benzo-1,2,3-thiadiazole derivatives
Dérivés de benzo-1,2,3-thiadiazole microbicides

(84) Benannte Vertragsstaaten:
**AT BE CH DE DK ES FR GB GR IT LI LU NL PT SE**

(30) Priorität: **06.03.1991 CH 66691**

(43) Veröffentlichungstag der Anmeldung:
**09.09.1992 Patentblatt 1992/37**

(73) Patentinhaber:
- **Novartis AG**
  **4058 Basel (CH)**
  Benannte Vertragsstaaten:
  **BE CH DE DK ES FR GB GR IT LI LU NL PT SE**
- **Novartis-Erfindungen Verwaltungsgesellschaft m.b.H.**
  **1235 Wien (AT)**
  Benannte Vertragsstaaten:
  **AT**

(72) Erfinder:
- **Kunz, Walter, Dr.**
  **CH-4104 Oberwil (CH)**
- **Schurter, Rolf, Dr.**
  **CH-4102 Binningen (CH)**

(74) Vertreter:
**Zumstein, Fritz, Dr. et al**
**Patentanwälte,**
**Dr. F. Zumstein,**
**Dipl.-Ing. F. Klingseisen,**
**Bräuhausstrasse 4**
**80331 München (DE)**

(56) Entgegenhaltungen:
**EP-A- 0 313 512** **GB-A- 1 176 799**

- **JOURNAL OF THE CHEMICAL SOCIETY, SECTION C 1971, LONDON GB Seiten 3994 - 3999; E. HADDOCK ET AL.: '1,2,3-Benzothiadiazoles. Part V. The Rearrangement of Diazonium Salts derived from 7-Aminobenzisothiazoles'**
- **JOURNAL OF CHEMICAL RESEARCH, MINIPRINT 1980, LONDON GB Seiten 2845 - 2866; K. CLARKE ET AL.: 'Condensed Isothiazoles. Part 7. Substitution Reactions of 3-Methyl-1,2-benzisothiazoles and Some of Its 4 and 5-Substituted Derivatives'**

Bemerkungen:
Die Akte enthält technische Angaben, die nach dem Eingang der Anmeldung eingereicht wurden und die nicht in dieser Patentschrift enthalten sind.

EP 0 502 473 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft teilweise neue substituierte Benzo-1,2,3-thiadiazol-Derivate der nachstehenden Formel I. sowie die Verwendung von Verbindungen der Formel I zum Schutz von Pflanzen gegen den Befall durch schädliche Mikroorganismen, insbesondere pflanzenschädigende Pilze.

Die erfindungsgemässen Mittel enthalten als Wirkstoffe Verbindungen der allgemeinen Formel I

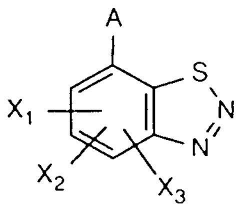

(I)

worin bedeuten:

$X_1$, $X_2$ und $X_3$ Wasserstoff,
A durch maximal 3 X-$C_1$-$C_4$-Alkylgruppen substituiertes $C_1$-$C_2$-Alkyl, durch 2 oder 3 Halogenatome substituiertes Methyl, durch Hydroxy und/oder bis zu 4 Halogenatome substituiertes Ethyl, unsubstituiertes oder bis zu 3 Halogenatome tragendes Vinyl; ferner Ethinyl, Propargyl, Formyl, Acetyl, ferner durch maximal 3 Halogenatome substituiertes Acetyl oder eine der Gruppen $C(R)=N-N(R_2)R_3$, $C(N=N-U_1)=N-NH-U_1$, $CH(R)-[N(R_1)]_n-N(R_2)R_3$, $C(R)(CN)OR_4$, $C(R)=N(O)_nR_3$, $CH(R)-O-N=C(R_1)R_2$, $CH(R)-O-N=C(CN)-CONH-R_5$, $C(R)_6=N(O)_nR$, $CH(R)-Y-E-R_3$, $CO-[C(OR)_2]_nQ$, $C(Q)=CH-OR$ oder T-Q;
worin ferner bedeuten:
n Null oder 1;
X und Y unabhängig voneinander Sauerstoff oder Schwefel;
R und $R_1$ unabhängig voneinander Wasserstoff oder $C_1$-$C_2$-Alkyl:
$R_2$ Wasserstoff, $C_1$-$C_8$-Alkyl, $C_3$-$C_6$-Alkenyl, $C_3$-$C_6$-Alkinyl, $C_3$-$C_7$-Cycloalkyl, Benzyl oder Cyano;
$R_3$ Wasserstoff, $C_1$-$C_6$-Alkyl, $C_3$-$C_6$-Alkenyl, $C_3$-$C_6$-Alkinyl, $C_3$-$C_7$-Cycloalkyl, Benzyl oder einen Arylrest U;
$R_4$ Wasserstoff, $C_1$-$C_6$-Alkyl, $Si(C_1$-$C_6$-$Alkyl)_3$ oder $OCOC_1$-$C_3$-Alkyl;
$R_5$ Wasserstoff oder $CONHR_1$;
$R_6$ Hydrazino, $N(R_1)R_2$ oder Q;
E CO oder $SO_2$;
U und $U_1$ unabhängig voneinander einen unsubstituierten oder durch Methyl, Methoxy, Halogen,Trifluormethyl, Nitro oder Cyano ein- oder mehrfach gleich oder verschieden substituierten Phenylrest;
T $C_1$-$C_2$-Alkylen, unabhängig voneinander durch Amino, Hydroxy oder Halogen substituiertes Methylen, unsubstituiertes oder durch Halogen oder Cyano substituiertes Ethenylen;
Q COXR oder Cyano.

Halogen bedeutet Fluor, Chlor, Brom oder Jod, bevorzugt Fluor und weiter in der Reihenfolge Chlor, Brom und Jod. Als Substituent in einzelnen Resten kann Halogen bis zu 3-fach vertreten sein.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende bevorzugte Gruppen dar: Methyl, Ethyl sowie die Isomeren von Propyl, Butyl, Pentyl oder Hexyl, wie z.B. Isopropyl, Isobutyl, tert.-Butyl, sek.-Butyl oder Isopentyl.

Alkenyl steht z.B. für Propenyl-(1), Allyl, Butenyl-(1), Butenyl-(2) oder Butenyl-(3) und Alkinyl bedeutet z.B. Propinyl-(2), Butinyl-(1) oder Pentinyl-(4).

Als Cycloalkyle seien z.B. genannt Cyclopropan, Cyclobutan, Cyclopentan, Cyclohexan oder Cycloheptan, vorzugsweise Cyclopropan, Cyclopentan und Cyclohexan.

Aufgrund ihrer besonderen pflanzenschützenden Eigenschaften und bevorzugten Verwendung lassen sich die Wirkstoffe der Formel I in folgende bevorzugte Gruppen einteilen:

1. Verbindungen worin bedeuten:

$X_1$, $X_2$ und $X_3$ Wasserstoff
A durch maximal 3 X-$C_1$-$C_4$-Alkylgruppen substituiertes $C_1$-$C_2$-Alkyl, durch 2 oder 3 Halogenatome substituiertes Methyl, durch Hydroxy und/oder bis zu 4 Halogenatome substituiertes Ethyl, unsubstituiertes oder bis zu 3 Halogenatome tragendes Vinyl; ferner Ethinyl, Propargyl, Formyl, Acetyl oder eine der Gruppen C(R)=N-$N(R_2)R_3$, $C(N=N-U_1)$=N-NH-U, CH(R)-$[N(R_1)]_n$-$N(R_2)R_3$, C(R)(CN)$OR_4$, C(R)=$N(O)_nR_3$, CH(R)-O-N=$C(R_1)R_2$, CH(R)-O-N=C(CN)-CONH-$R_5$, $C(R_6)$=N-$(O)_n$R, CH(R)-Y-E-$R_3$, CO-$[C(OR)_2]_n$COXR, C(Q)=CH-OR oder T-Q;

worin ferner bedeuten:

n Null oder 1;
X und Y Sauerstoff;
R und $R_1$ unabhängig voneinander Wasserstoff oder $C_1$-$C_2$-Alkyl;
$R_2$ Wasserstoff, $C_1$-$C_4$-Alkyl, $C_3$-$C_4$-Alkenyl, $C_3$-$C_4$-Alkinyl, $C_3$-$C_6$-Cycloalkyl oder Benzyl;
$R_3$ Wasserstoff, $C_1$-$C_4$-Alkyl, $C_3$-$C_4$-Alkenyl, $C_3$-$C_4$-Alkinyl, $C_3$-$C_6$-Cycloalkyl oder einen durch Methyl, Halogen oder Trifluormethyl gleich oder verschieden substituierten Phenylrest;
$R_4$Wasserstoff, $C_1$-$C_3$-Alkyl oder Si($C_1$-$C_2$-$Alkyl)_3$;
$R_5$ Wasserstoff oder CONH$C_1$-$C_3$-Alkyl;
$R_6$ N(R)$R_1$, Hydrazino oder Q;
U, $U_1$ unabhängig voneinander einen durch Methyl, Halogen, Nitro oder Trifluormethyl substituierten Phenylrest;
E CO;
T Methylen, durch Amino substituiertes Methylen oder Ethenylen;
Q COOR oder Cyano.

2. Verbindungen, worin bedeuten:

$X_1$, $X_2$ und $X_3$ Wasserstoff;
A durch maximal 2 X-$C_1$-$C_4$-Alkylgruppen substituiertes Methyl, durch 2 oder 3 Fluor- oder Chloratome substituiertes Methyl, durch Hydroxy oder 1 bis 4 Halogenatome substituiertes Ethyl, oder Formyl, Acetyl oder eine der Gruppen C(R)=N-$N(R_2)R_3$, CH(R)-$[N(R_1)]_n$-$N(R_2)R_3$, C(R)(CN)$OR_4$, C(R)=N-$R_3$, CH(R)-O-N=$C(R_1)R_2$, CH(R)-O-N=C(CN)-CONH$R_5$, $C(R_6)$=$N(O)_n$R, CH(R)-Y-E-$R_3$, CO$[C(OR)_2]_n$COXR, C(COOR)=CH-OR oder T-Q;
worin ferner bedeuten:
n 1;
X Sauerstoff;
R und $R_1$ unabhängig voneinander Wasserstoff oder Methyl;
$R_2$ Wasserstoff, $C_1$-$C_2$-Alkyl, Allyl, Propargyl, Cyclopropyl oder Benzyl;
$R_3$ Wasserstoff, $C_1$-$C_2$-Alkyl, Allyl, Propargyl, Cyclopropyl oder einen durch Methyl, Fluor, Chlor oder Trifluormethyl gleich oder verschieden subtituierten Phenylrest;
$R_4$ Wasserstoff, $C_1$-$C_2$-Alkyl oder $Si(CH_3)_3$;
$R_5$ Wasserstoff oder CONH-$C_1$-$C_2$-Alkyl;
$R_6$ Amino oder Q;
Y Sauerstoff;
E CO;
T Methylen oder Cyano;
Q $COOCH_3$ oder CN.

3. Verbindungen, worin bedeuten:

$X_1$, $X_2$ und $X_3$ Wasserstoff;
A durch maximal 2 X-$C_1$-$C_2$-Alkylgruppen substituiertes Methyl, durch 2 oder 3 Fluoratome substituiertes Methyl, durch Hydroxy oder Chlor substituiertes Ethyl, oder Formyl, oder eine der Gruppen C(R)=N-$N(R_2)R_3$, CH(R)-$[N(R_1)]_n$-$N(R_2)R_3$, C(R)(CN)$OR_4$, C(R)=$N(O)_nR_3$, CH(R)-O-N=$C(R_1)R_2$, $C(R_6)$=N-OR, CH(R)-Z-E-$R_3$, CO$[C(OR)_2]_n$COXR oder T-Q;
worin ferner bedeuten:
n 1;
X Sauerstoff;
R und $R_1$ unabhängig voneinander Wasserstoff oder Methyl;
$R_2$ Wasserstoff, Methyl, Allyl, Cyclopropyl oder Benzyl;
$R_3$ Wasserstoff, Methyl, Allyl, Cyclopropyl oder einen durch Methyl, Fluor, Chlor oder Trifluormethyl gleich oder verschieden subtituierten Phenylrest;

$R_4$ Wasserstoff, Methyl oder $Si(CH_3)_3$;
$R_5$ Wasserstoff oder $CONH-CH_2-CH_3$;
$R_6$ Amino;
Y Sauerstoff;
E CO;
T Methylen;
Q Cyano oder $COOCH_3$.

Folgende Wirkstoffe der Formel I zeichnen sich durch besonders vorteilhafte pflanzenschützende Eigenschaften aus:

7-Formyl-1,2,3-benzothiadiazol; (vorbekannt)
7-Acetoxymethyl-1,2,3-benzothiadiazol;
7-[Methoxyimino-(2-cyanoacetamidyl)]-1,2,3-benzothiadiazol;
7-(N-Methoxyimino-methyl)-1,2,3-benzothiadiazol;
7-(N-Methoxyimino-hydroxymethyl)-1,2,3-benzothiadiazol;
7-Methoxymethyl-1,2,3-benzothiadiazol;
3-(7-Benzo-1,2,3-thiadiazol)-acrylsäure;
7-Cyanomethyl-1,2,3-benzothiadiazol;
7-Trichlormethyl-1,2,3-benzothiadiazol;
7-Dichlormethyl-1,2,3-benzothiadiazol;
Benz-1,2,3-thiadiazol-7-(N-hydroxycarboximid-amid);
Benz-1,2,3-thiadiazol-7-(N-methoxyhydroxamsäure);
2-(Benz-1,2,3-thiadiazolyl)-2-hydroxyiminoacetonitril;
5-Fluor-benz-1,2,3-thiadiazol-7-carbaldehyd;
6-Fluor-benz-1,2,3-thiadiazol-7-carbaldehyd;
4-Fluor-benz-1,2,3-thiadiazol-7-carbaldehyd;
N,N-Diphenyl-C-[benz-1,2,3-thiadiazol-7'yl]formazan;
7-Acetyl-benz-1,2,3-thiadiazol; (vorbekannt)
7-(Bromacetyl)benz-1,2,3-thiadiazol.

Verbindungen, die unter die Formel I fallen, sind neu mit Ausnahme von:

7-Formyl-1,2,3-benzothiadiazol;
7-Acetyl-1,2,3-benzothiadiazol;
6-Chlor-7-formyl-1,2,3-benzothiadiazol;
6-Methylthio-7-formyl-1,2,3-benzothiadiazol;
4-Brom-6-chlor-7-formyl-1,2,3-benzothiadiazol;
6-Methoxy-7-formyl-1,2,3-benzothiadiazol;
7-Hydroxyimino-1,2,3-benzothiadiazol;
6-Methoxy-7-oximino-1,2,3-benzothiadiazol.
7-Dibromacetyl-1,2,3-benzothiadiazol.

Die neuen Verbindungen stellen einen besonderen Bestandteil der vorliegenden Erfindung dar.

Besonders bevorzugt sind die neuen Verbindungen aus der Gruppe:

7-Acetoxymethyl-1,2,3-benzothiadiazol;
7-[Methoxyimino-(2-cyanoacetamyl)]-1,2,3-benzothiadiazol;
7-(N-Methoxyimino-methyl)-1,2,3-benzothiadiazol;
7-(N-Methoxyimino-hydroxymethyl)-1,2,3-benzothiadiazol;
7-Methoxymethyl-1,2,3-benzothiadiazol;
3-(7-Benzo-1,2,3-thiadiazol)-acrylsäure;
7-Cyanomethyl-1,2,3-benzothiadiazol;
7-Trichlormethyl-1,2,3-benzothiadiazol;
7-Dichlomlethyl-1,2,3-benzothiadiazol;
Benz-1,2,3-thiadiazol-7-(N-hydroxycarboximid-amid);
Benz-1,2,3-thiadiazol-7-(N-methoxyhydroxamsäure);
2-(Benz-1,2,3-thiadiazolyl)-2-hydroxyiminoacetonitril;

5-Fluor-benz-1,2,3,-thiadiazol-7-carbaldehyd;
6-Fluor-benz-1,2,3-thiadiazol-7-carbaldehyd;
4-Fluor-benz-1,2,3-thiadiazol-7-carbaldehyd;
N,N-Diphenyl-C-[benz-1,2,3-thiadiazol-7'yl]formazan;
7-(Bromacetyl)-benz-1,2,3-thiadiazol.

Benz-1,2,3-thiadiazole mit pflanzenmikrobizider und pflanzenimmunisierender Wirkung sind in EP-A-313,512 beschrieben.

Die vorstehend als nicht neu bezeichneten Verbindungen sind aus folgender Literatur bekannt: J. Chem. Res. 1980, 197 und 2845; J. Chem. Soc. 1971, 3994; der britischen Patentschrift Nr. 1 176 799; der niederländischen Patentanmeldung 67,16077, der deutschen Offenlegungsschrift 1,695,786.

Die Verbindungen der Formel I werden wie folgt hergestellt:

1.1 Nitrierung einer Verbindung der Formel II

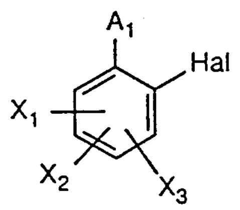

(II)

mit $HNO_3$ mit oder ohne Zusatz von $H_2SO_4$ und/oder Lösungsmitteln, wie $CH_2Cl_2$, bei Temperaturen von -20° bis 80°C zu einer Verbindung der Formel III

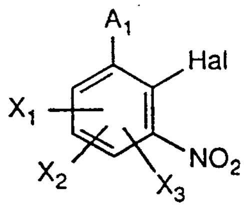

(III);

1.2 Umsetzung einer Verbindung der Formel III mit einer Verbindung der Formel IIIa

MSL (IIIa)

in inerten Lösungsmiteln mit oder ohne Base bei Temperaturen von -5° bis 50°C zu einer Verbindung der Formel IV

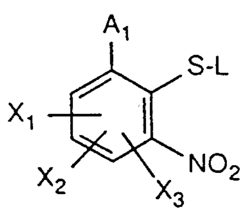

(IV);

1.3 Reduktion einer Verbindung der Formel IV mit Wasserstoff in Gegenwart eines Katalysators oder mit Eisenpulver in Essigsäure in inerten Lösungsmitteln bei Temperaturen von 0 bis 120°C zu einer Verbindung der Formel V

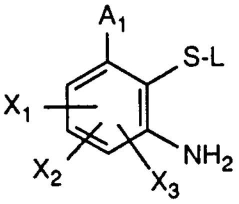

(V);

1.4 Diazotierung einer Verbindung der Formel V mit $NaNO_2$ in Gegenwart einer Säure in Wasser und zusätzlich mit inerten Lösungsmitteln oder ohne diese bei Temperaturen von -30° bis 50°C zu einer Verbindung der Formel Ia

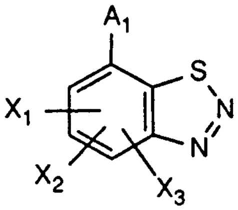

(Ia),

wobei in den vorstehenden Formeln $A_1$ die unter Formel I genannten Reste A mit Ausnahme derjenigen, welche freie Hydroxy-, Thiol- oder NH-Gruppen enthalten, sowie der in wässrig-saurem Milieu unbeständigen Gruppen, z.B. der Acetale oder Ketale, bedeuten und $X_1$, $X_2$ und $X_3$ die unter Formel I angegebenen Bedeutungen besitzen;
2.1 Verbindungen der Formeln Ib, Ic und Id

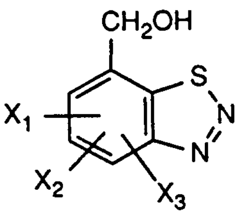

(Ib)

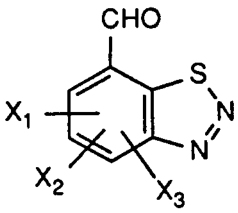

(Ic)

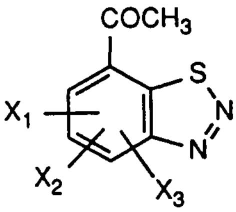

(Id)

werden hergestellt:

2.1.1. durch Umsetzung einer Verbindung der Formel VI

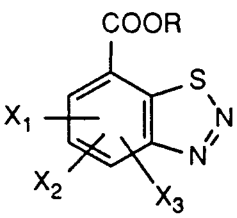

(VI)

a) mit einem Reduktionsmittel in inerten Lösungsmitteln bei -20° bis 120°C, bevorzugt 0° bis 80°C, z.B.
b) mit Natriumborhydrid in inerten Lösungsmitteln, wie z.B. Tetrahydrofuran oder Dioxan, in Gegenwart

von Wasserbei -20° bis 100°C, bevorzugt 0° bis 80°C, zu einer Verbindung der Formel Ib; oder durch Umsetzung des Säurechlorids der Formel VII'

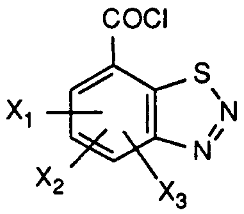

(VII')

nach Rosenmund mit Wasserstoff in einem inerten Lösungsmittel, wie z.B. Tetrahydrofuran, in Gegenwart einer Base, z.B. Lutidin, mittels eines Katalysators, z.B. Palladium auf Bariumsulfat, bei -20° bis 120°C, bevorzugt 0° bis 120°C, ferner bevorzugt 0° bis 80°C, zu einer Verbindung der Formel Ib, und

2.1.2 durch Oxidation einer Verbindung der Formel Ib mit einem Oxidationsmittel, z.B. Mangandioxid, in einem inerten Lösungsmittel, wie z.B. Chloroform, oder Cerammoniumnitrat in einem Essigsäure/Wasser-Gemisch bei 0° bis 110°C, bevorzugt 20° bis 100°C, zu einer Verbindung der Formel Ic.
2.1.3 durch Umsetzung eines Nitrils der Formel V

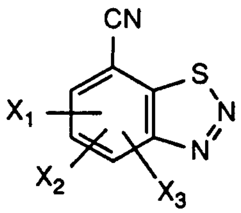

(V)

oder eines Hydroxamsäure-Derivats der Formel VIII

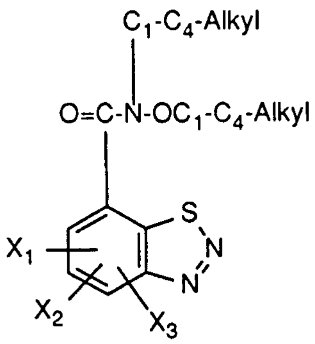

(VIII)

miteinem Grignard-Reagens, $CH_3Mg$-Halogen, in einem inerten Lösungsmittel, wie z.B. einem offenkettigen oder cyclischen Ether (z.B. Tetrahydrofuran oder Dioxan), bei - 50° bis 130°C, bevorzugt -10° bis 80°C, zu einer Verbindung der Formel Id, wobei die in den vorstehend beschriebenen Formeln auftretenden Reste $X_1$, $X_2$ und $X_3$ die unter Formel I angegebenen Bedeutungen besitzen und R in Formel VI Wasserstoff oder Methyl darstellt;

3.1 Verbindungen der Formel Ie

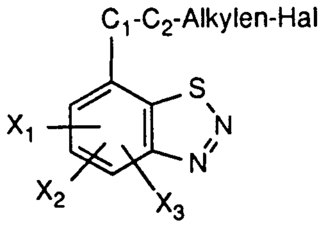

(Ie)

werden hergestellt:
durch Umsetzung einer Verbindung der Formel Ib'

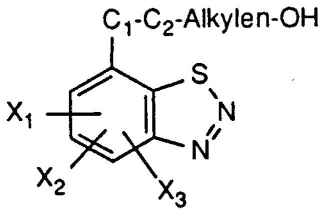

(Ib')

mit einem Halogenierungsmittel, wie z.B. Thionylhalogenid, in inerten Lösungsmitteln, wie z.B. Dichlormethan, in Gegenwart einer Base, wie z.B. Pyridin, bei - 20° bis 150°C, wobei Hal Halogen darstellt und die Reste $X_1$, $X_2$ und $X_3$ die unter Formel I angegebenen Bedeutungen besitzen;

3.2 Verbindungen der Formel If

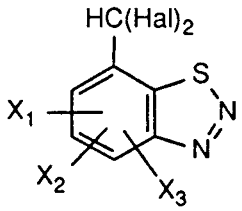

(If)

werden hergestellt:
durch Umsetzung einer Verbindung der Formel Ic

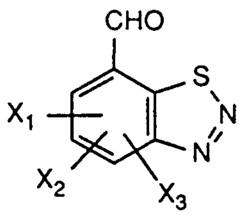

(Ic)

mit einem Halogenierungsmittel, wie z.B. Thionylhalogenid, in inerten Lösungsmitteln, wie z.B. Dichlormethan, in Gegenwart einer Base, wie z.B. Pyridin, bei - 20° bis 150°C, wobei Hal Halogen darstellt und die Reste $X_1$, $X_2$ und $X_3$ die unter Formel I angegebenen Bedeutungen besitzen;

3.3 Verbindungen der Formel Ig

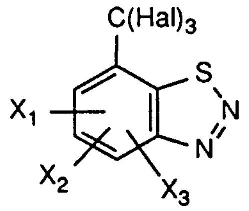

(Ig)

werden hergestellt:

3.3.1 durch Umsetzung einer Verbindung der Formel VI'

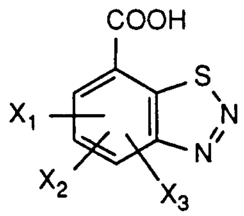

(VI')

mit einem Halogenierungsmittel, wie z.B. Phosphorpentahalogenid, Phenylphosphoroxydichlorid oder Phenylenoxyphosphordichlorid, in inerten Lösungsmittel oder ohne diese bei 20° bis 250°C, bevorzugt 80° bis 200°C; oder

3.3.2 durch Umsetzung einer Verbindung der Formel Ig mit Schwefeltetrafluorid in Gegenwart von Fluorwasserstoffsäure in einem Autoclaven bei 0° bis 250°C, wobei Hal Halogen und R Wasserstoff oder Methyl bedeuten und die Reste $X_1$, $X_2$ und $X_3$ die unter Formel I angegebenen Bedeutungen besitzen;

3.4 Verbindungen der Formel Ih

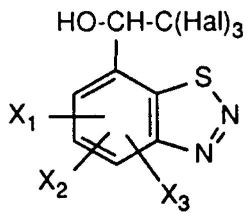

(Ih)

werden hergestellt durch Umsetzung einer Verbindung der Formel Ic mit 2,2,2-Trihalogenessigsäure in dipolar aprotischen Lösungsmitteln, wie z.B. Hexamethylphosphorsäuretriamid oder Dimethylsulfoxid, bei 0° bis 150°C, bevorzugt 20° bis 100°C; und

3.5 Verbindungen der Formel Ii

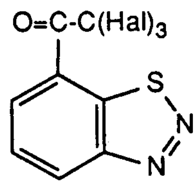

(Ii)

werden hergestellt:

3.5.1 durch Umsetzung einer Verbindung der Formel Id mit einem Halogenierungsmittel, z.B. elementaren, Halogen oder Sulfonylhalogenid, mit oder ohne Zusatz von Radikalen oder

3.5.2 durch Oxidation einer Verbindung der Formel Ih mit einem Oxidationsmittel, wie z.B. $MnO_2$ oder Pyridinchlorchromat, in einem inerten Lösungsmittel, wie Chloroform, bei 20° bis 150°C; wobei in den vorstehenden Formeln Hal Fluor, Chlor, Brom oder Jod bedeutet;

4. Verbindungen der Formeln $Ik_1$ und $Ik_2$

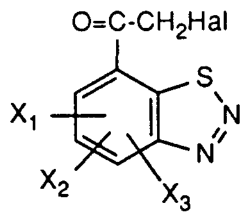

($Ik_1$)

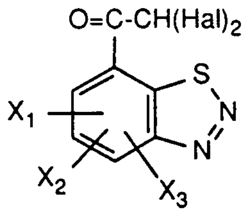

($Ik_2$)

werden hergestellt durch Umsetzung jeweils einer Verbindung der Formel Id mit stöchiometrisch abgestimmten Mengen eines Halogenierungsmittel, z.B. elementarem Halogen oder Sulfonylhalogenid, mit oder ohne Zusatz von Radikalen, wobei Hal Halogen bedeutet und $X_1$, $X_2$ und $X_3$ die unter Fomel I angegebenen Bedeutungen besitzen; und
5.1 Verbindungen der Formel $Il_1$

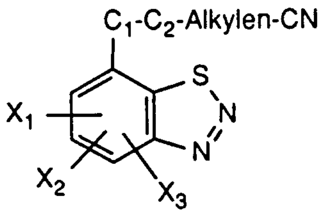

($Il_1$)

werden hergestellt durch Umsetzung von Verbindungen der Formel Ie mit Cyanidverbindungen der Formel MCN in inerten Lösungsmitteln bei -10° bis 120°C, wobei die Reaktion auch im 2-Phasensystem, z.B. in $CHCl_3/H_2O$, in Gegenwart eines quaternären Ammoniumsalzes, wie z.B. Tetrabutylammoniumchlorid oder -jodid, durchgeführt werden kann; und
5.2 Verbindungen der Formeln $Il_2$ und $Il_3$

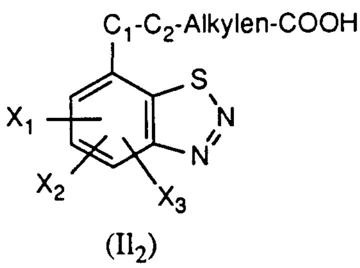

($Il_2$)

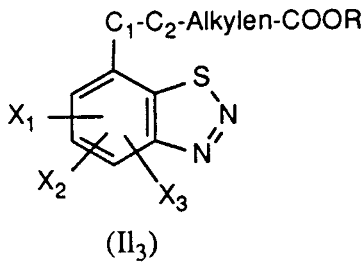

($Il_3$)

werden hergestellt durch Hydrolyse von Nitrilen der Formel $Il_1$ zu den Verbindungen der Formel $Il_2$ und deren Veresterung mit Verbindungen der Formel ROH, wobei in den vorstehenden Formeln M ein Alkalimetall oder Ammonium bedeutet, R $C_1$-$C_4$-Alkyl darstellt und $X_1$, $X_2$ und $X_3$ die unter Formel I angegebenen Bedeutungen besitzen;
6.1 Verbindungen der Formeln $Im_1$ und $Im_2$

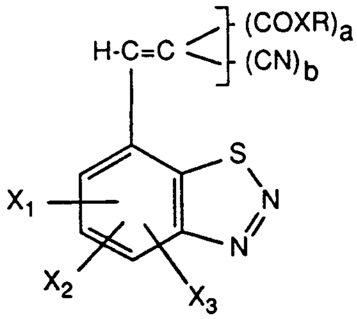

(Im1)

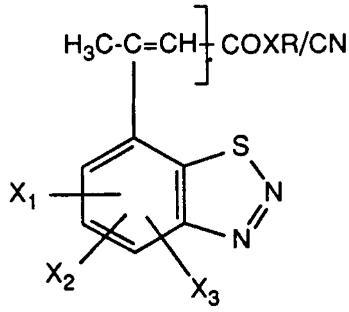

(Im2)

werden hergestellt durch Kondensation von Verbindungen der Formel Ic oder Id

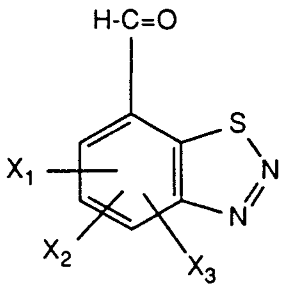

(Ic)

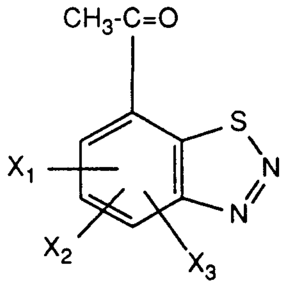

(Id)

mit Verbindungen der Formel

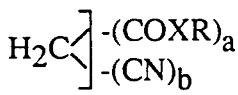

in Gegenwart von Katalysatoren, wie z.B. Ammoniumacetat oder niederen organischen Carbonsäuren, sowie Basen, wie z.B. Pyridin oder Piperidin, mit oder ohne Zusatz von Lösungsmitteln, z.B. Toluol, mit oder ohne Abscheidung des gebildeten Wassers, z.B. durch azeotrope Destillation oder durch Verwendung eines Molekularsiebs, wobei (a+b) = 2 und a = (2-b) sind und R, X, $X_1$, $X_2$ und $X_3$ die unter Formel I angegebenen Bedeutungen besitzen.

Falls gewünscht, kann bei Verwendung eines geeigneten Malonsäure- oder Cyanessigsäure-Derivats als aktive Methylenkomponente eine Decarboxylierung (Knoevenagel-Reaktion) erzielt werden. Diese kann in einem Reaktionsschritt oder nach Hydrolyse einer COXR- oder CN-Gruppe zur COOH-Gruppe in einem anderen Reaktionsschritt durch Erhitzen auf 30° bis 300°C mit oder ohne inerter Lösungsmittel erfolgen; und
7.1 Verbindungen der Formel $In_1$

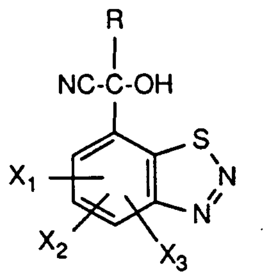

$(In_1)$

werden hergestellt durch Umsetzung von Verbindungen der Formeln Ic und Id mit Alkalicyanid oder Cyanwasserstoff in einem inerten Lösungsmittel, wie z.B. Tetrahydrofuran oder Methanol, mit oder ohne Zusatz von Natriumhydrogensulfit bei -20° bis 140°C, bevorzugt 0° bis 90°C, oder durch Hydrolyse von Silylestern der Formel $In_2$

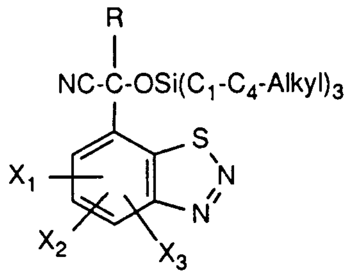

$(In_2)$

mit verdünnten Mineralsäuren, z.B. HCl, in inerten Lösungsmittel bei - 20° bis 120°C;

7.2 Verbindungen der Formel $In_2$ werden hergestellt durch Umsetzung von Aldehyden der Formel Ic oder von Ketonen der Formel Id mit Trialkyl-silylcyanid in inerten Lösungsmitteln, wie z.B. Tetrahydrofuran, Dioxan, Chloroform oder Dichlormethan, bevorzugt unter Zusatz katalytischer Mengen eines Metallsalzes, wie z.B. $ZnI_2$, bei - 30° bis 130°C, bevorzugt -10°C bis 100°C;

7.3 Verbindungen der Formel $In_3$

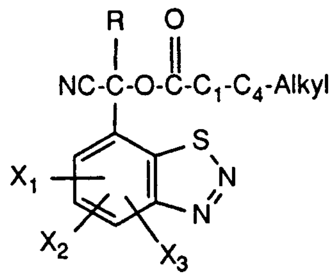

$(In_3)$

werden hergestellt durch Umsetzung von Verbindungen der Formel $In_1$ mit Säurechloriden oder -anhydriden in Gegenwart einer Base, wie z.B. Triethylamin, und eines Katalysators, wie z.B. 4-Dimethylaminopyridin; und

7.4 Verbindungen der Formel $In_4$

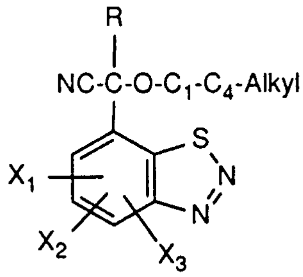

(In$_4$)

werden hergestellt:

7.4.1 durch Umsetzung von Verbindungen der Formel In$_1$ mit einem Alkylierungsmittel, wie z.B. eines $C_1$-$C_4$-Alkylhalogenids, bevorzugt eines $C_1$-$C_4$-Alkyljodids, in Gegenwart einer Base, wie z.B. Triethylamin oder Alkali- oder Erdalkalicarbonats, in inerten Lösungsmitteln bei -20° bis 100°C; oder

7.4.2 durch Umsetzung von Acetalen der Formel In$_5$

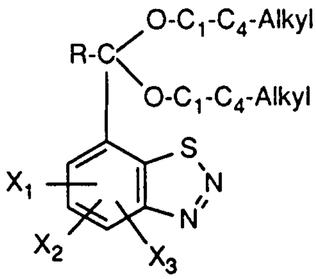

(In$_5$)

mit Tri($C_1$-$C_4$-Alkyl)silylcyaniden in Gegenwart einer Lewissäure, wie z.B. Bortrifluoridetherat, in inerten Lösungsmitteln bei -20° bis 150°C;

8.1 Verbindungen der Formel Io$_1$

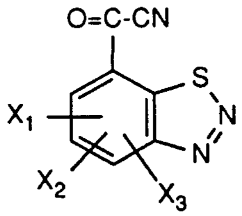

(Io$_1$)

werden hergestellt durch Umsetzung von Verbindungen der Formel IX

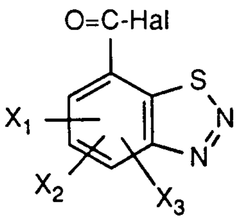

(IX)

mit Metallsalzen der Formel $M^{m\oplus}(CN)m$, worin M ein Metallkation, z.B. $Na^{\oplus}$, $K^{\oplus}$, $Ag^{\oplus}$ oder $Pb^{2\oplus}$, ist, in inerten Lösungsmitteln bei -20° bis 120°C, wobei m 1 oder 2 bedeutet und Hal für Halogen steht;

8.2 Verbindungen der Formel $Io_2$

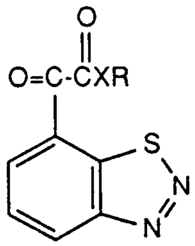

($Io_2$)

werden hergestellt:

8.2.1 durch Oxidation entsprechender α-Hydroxyverbindungen, mit einem Oxidationsmittel, z.B. Mangandioxid oder Pyridinchlorchromat; oder

8.2.2 durch saure Hydrolyse von Verbindungen der Formel Ii oder von Verbindungen der Formel $Io_1$ mit verdünnten Basen, z.B. Alkali- oder Erdalkalihydroxid-oder -carbonatlösungen, mit oder ohne Zusatz von inerten Lösungsmitteln bei -10° bis 150°C, bevorzugt 20° bis 100°C; oder

8.2.3 durch Umsetzung von Verbindungen der Formel Iq

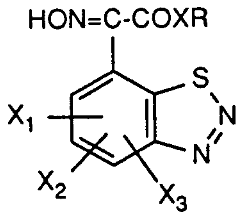

(Iq)

mit überschüssigem wässrigem Formaldehyd oder mit Aceton in Gegenwart einer Säure, z.B. HCl, und mit oder ohne Zusatz eines Oxidationsmittels, wie z.B. Dichlor-dicyanobenzo-chinon, Selendioxid oder Pyridinchlorchromat;

8.2.4 durch Umsetzung von Verbindungen der Formel $Ix_2$

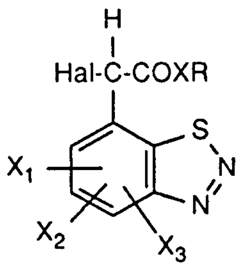

($Ix_2$)

mit Alkaliazid in inerten Lösungsmitteln, z.B. Dimethylformamid, bei - 20° bis 50°C zu Verbindungen der Formel

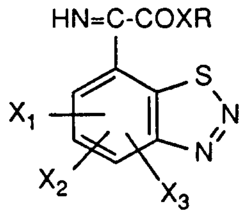

und anschliessender wässriger Hydrolyse mit oder ohne Zusatz eines Katalysators, z.B. $CuSO_4$; und

8.3 Verbindungen der Formel $Io_3$

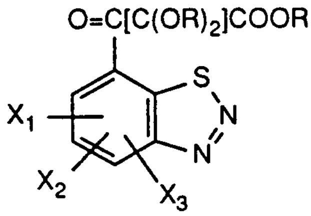

($Io_3$)

werden hergestellt durch Umsetzung von Verbindungen der Formel VII

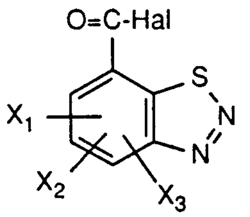

(VII)

mit einem Tetraalkoxyethylen der Formel

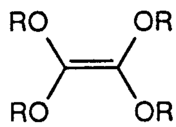

in inerten Lösungsmitteln, z.B. Toluol, bei 20° bis 220°C; wobei Hal Halogen, X Sauerstoff oder Schwefel und R $C_2$-$C_4$-Alkyl bedeuten und $X_1$, $X_2$ und $X_3$ die unter Formel I angegebenen Bedeutungen besitzen;
9.1 Verbindungen der Formel Ip

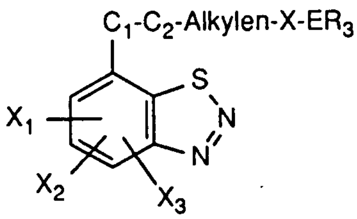

(Ip)

werden hergestellt:

9.1.1 durch Umsetzung von Verbindungen der Formel Ib mit Säurechloriden der Formel $R_3$-E-Hal oder Säureanhydriden der Formel $(R_3\text{-}E)_2O$ in Gegenwart einer Base, z.B. Triethylamin, mit oder ohne Zusatz eines Katalysators, z.B. 4-Dimethylaminopyridin, in einem inerten Lösungsmittel, z.B. Dichlormethan, bei -20° bis 150°C; oder

9.1.2 durch Umsetzung von Verbindungen der Formel Ie mit Alkalisalzen der Formel $R_3COXM$ in einem dipolar aprotischen Lösungsmittel, wie z.B. N,N-Dimethylformamid oder Dimethylsulfoxid, bei -20° bis 150°C; wobei X, E und $R_3$ sowie $X_1$, $X_2$ und $X_3$ die unter Formel I angegebenen Bedeutungen besitzen;

10.1 Verbindungen der Formel $Iq_1$

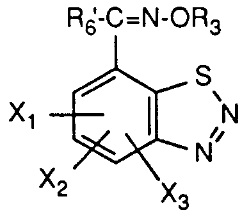

($Iq_1$)

werden hergestellt durch Umsetzung von Verbindungen der Formel Ic, Id, Ii, Ik und $Io_2$ mit Verbindungen der Formel $NH_2OR_3$ oder deren Hydrochlorid in einem protischen Lösungsmittel mit oder ohne Zusatz einer Base, wie z.B. Alkalicarbonat, -hydroxid oder -oxid, oder mit oder ohne Zusatz einer Säure, wie Essigsäure, bei -10° bis 120°C; wobei $R_6'$ Wasserstoff, Methyl, COXR oder CN bedeutet und X, R und $R_3$ sowie $X_1$, $X_2$ und $X_3$ die unter Formel I angegebenen Bedeutungen besitzen; und

10.2 Verbindungen der Formel $Iq_2$

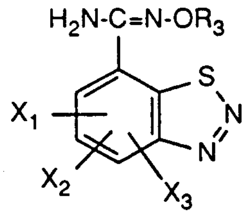

($Iq_2$)

werden hergestellt durch Umsetzung von Verbindungen der Formel V mit Verbindungen der Formel $H_2N\text{-}OR_3$ in einem inerten Lösungsmittel, z.B. Ethanol, bei - 20° bis 120°C; und

10.3 Verbindungen der Formel $Iq_3$

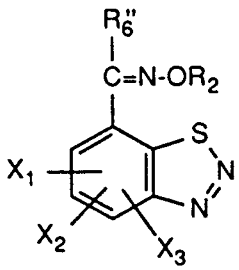

($Iq_3$)

werden hergestellt durch Umsetzung von Verbindungen der Formel $II_1$, $II_2$ und $II_3$ mit salpetriger Säure oder einem Alkylester der salpetrigen Säure, z.B. iso-Amylnitrit, in Gegenwart einer Base, wie z.B. eines Alkalihydroxids oder Alkalialkoholats, bei - 30° bis 100°C; wobei Verbindungen der Formel $Iq_3$, in welcher $R_2$ Wasserstoff darstellt, nachträglich in Gegenwart einer Base, wie z.B. Natriumhydrid, Kalium-tert. butylat, Alkalihydroxid oder -carbonat, in einem inerten Lösungsmittel bei -20° bis 100°C mit Verbindungen der Formel $R_2$-Hal verethert werden können, undwobei $R_6''$ $CH_3$, COXR oder CN bedeutet, R $C_1$-$C_4$-Alkyl darstellt und $R_2$, $X_1$, $X_2$ und $X_3$ die unter Formel I angegebenen Bedeutungen besitzen; und

10.4 Verbindungen der Formel $Iq_4$

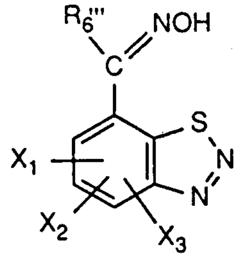

($Iq_4$)

worin $R_6'''$ für Hydrazino oder $NR(R_1)$ steht, werden aus den Benzhydroxamsäurechloriden Iq'

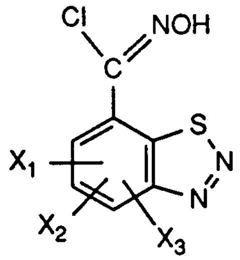

(Iq')

mit einem Amin der Formel $NR(R_1)$ oder Hydrazin in einem inerten Lösungsmittel bei -40° bis +100°C hergestellt. Benzhydroxamsäurechloride der Formel Iq' werden durch Chlorierung der entsprechenden Aldoxime mit einem Chlorierungsmittel z.B. $Cl_2$ in einem geeigneten Lösungsmittel wie verdünnte Chlorwasserstoffsäure bei -60° bis +50°C erhalten.

11.1 Verbindungen der Formel $Ir_1$

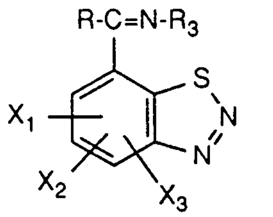

($Ir_1$)

werden hergestellt durch Umsetzung von Verbindungen der Formeln Ic oder Id mit Verbindungen der Formel $H_2N-R_3$ in aprotischen oder protischen Lösungsmitteln mit oder ohne Zusatz einer anorganischen oder organischen Säure, wie z.B. Eisessig, p-Toluolsulfonsäure oder Schwefelsäure, sowie mit oder ohne Durchführung einer azeotropen Destillation zur Entfernung des gebildeten Reaktionswassers oder durch Adsorption desselben an ein Molekularsieb bei 0° bis 150°C; und

11.2 Verbindungen der Formel $Ir_2$

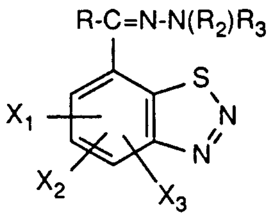

($Ir_2$)

werden hergestellt durch Umsetzung von Verbindungen der Formel Ic oder Id mit Verbindungen der Formel $H_2N-$

$N(R_2)R_3$ in aprotischen oder protischen Lösungsmitteln mit oder ohne Zusatz einer anorganischen oder organischen Säure, wie z.B. Eisessig, p-Toluolsulfonsäure oder Schwefelsäure, sowie mit oder ohne Durchführung einer azeotropen Destillation zur Entfernung des gebildeten Reaktionswassers oder durch Adsorption desselben an ein Molekularsieb bei 0° bis 150°C; wobei R, $R_1$, $R_2$ und $R_3$ sowie $X_1$, $X_2$ und $X_3$ die unter Formel I angegebenen Bedeutungen besitzen.

11.3 Verbindungen der Formel $Ir_3$

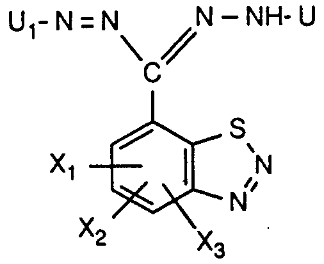

($Ir_3$)

werden durch Kupplung von Phenyldiazoniumsalzen $U_1$-$N_2^{\oplus}$ an Phenylhydrazone des Typs ($Ir_2$)' in wässriger alkoholischer Lösung in schwach saurem oder basischem Milieu bei Temperatur zwischen -20° und +30°C hergestellt. PH-Werte ≥ 3 während der Kupplungsreaktion können z.B. durch Zusätze von inerten Pyridin-Basen (Pyridin, Collidin) oder Alkalihydroxide oder -oxide eingestellt werden.

12.1 Verbindungen der Formeln $Is_1$ und $Is_2$

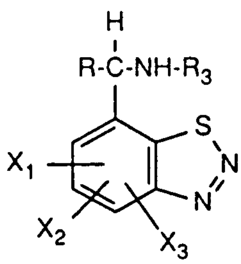

($Is_1$)

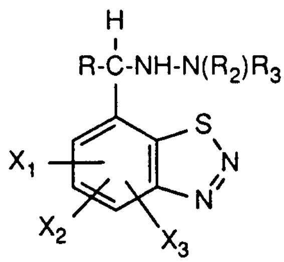

($Is_2$)

werden hergestellt entweder durch Reduktion der Verbindungen der Formeln $Ir_1$ bzw. $Ir_2$, z.B. durch katalytische Hydrierung in Gegenwart eines Metallkatalysators oder mittels eines komplexen Hydrids, und in bevorzugter Ausführungsform durch Umsetzung von Verbindungen der Formel

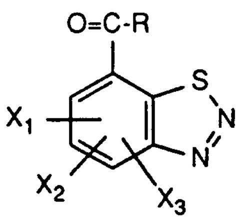

mit Verbindungen der Formeln $HN(R_2)R_3$ und $HN(R_1)$-$N(R_2)R_3$ in Gegenwart von Natriumcyanoborhydrid in verdünnter Essigsäure bei 0° bis 120°C; wobei $R_1$, $R_2$ und $R_3$ sowie $X_1$, $X_2$ und $X_3$ die unter Formel I angegebenen Bedeutungen besitzen; und

13.1 Verbindungen der Formeln $It_1$ und $It_2$

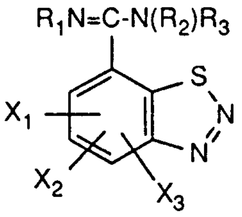

(It1)

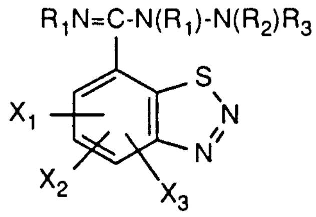

(It2)

werden hergestellt:

13.1.1 durch Umsetzung von Verbindungen der Formel V mit Verbindungen der Formel ROH in Gegenwart einer wasserfreien Säure, z.B. gasförmiger HCl, bei - 20° bis 90°C zu Verbindungen der Formel IX

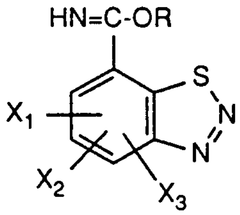

(IX)

und anschliessender Umsetzung der Verbindungen der Formel IX mit Verbindungen der Formeln $HN(R_2)R_3$ und $HN(R_1)\text{-}N(R_2)R_3$ in inerten Lösungsmitteln bei - 20° bis 120°C; oder

13.1.2 durch Umsetzung von Verbindungen der Formel X

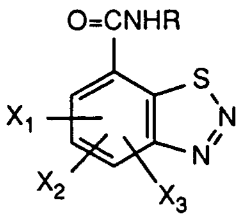

(X)

mit $PCl_5$ oder $CCl_4$ in Gegenwart von $P(Phenyl)_3$ und $CH_3CN$ in inerten Lösungsmitteln, wie z.B. Toluol oder Acetonitril, bei -50° bis 150°C, bevorzugt -20° bis 90°C, zu Verbindungen der Formel XI

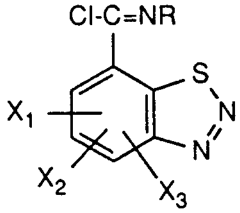

(XI)

und anschliessender Umsetzung von Verbindungen der Formel XI mit Verbindungen der Formeln $HN(R_2)R_3$ und $HN(R_1)\text{-}N(R_2)R_3$ in inerten Lösungsmitteln bei - 20° bis 120°C; wobei R, $R_1$, $R_2$ und $R_3$ sowie $X_1$, $X_2$ und $X_3$ die unter Formel I angegebenen Bedeutungen besitzen;

14.1 Verbindungen der Formeln $Iu_1$, $Iu_2$ und $Iu_3$

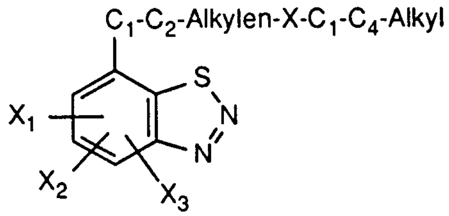

$(Iu_1)$

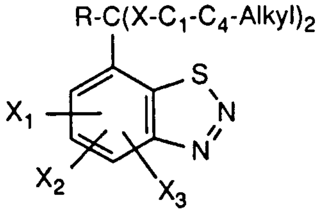

$(Iu_2)$

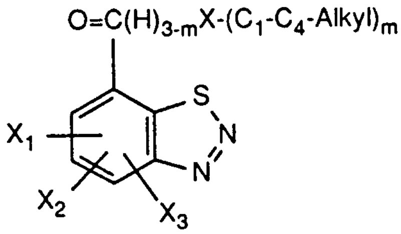

$(Iu_3)$

werden hergestellt durch Umsetzung von Verbindungen der Formeln Ie, If, Ig, $Ik_1$, $Ik_2$ und $Ik_3$

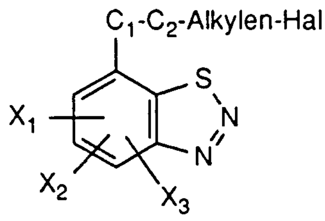

(Ie)

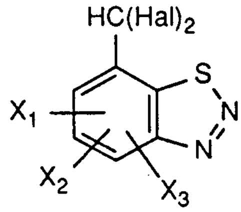

(If)

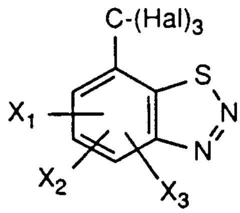

(Ig)

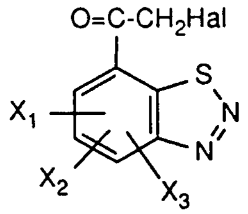

($Ik_1$)

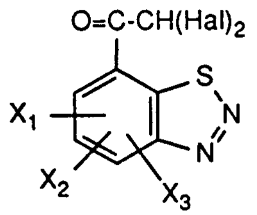

($Ik_2$)

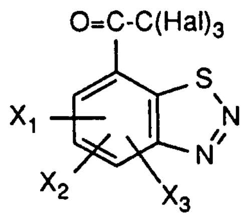

($Ik_3$)

mit Verbindungen der Formel H-X-$C_1$-$C_4$-Alkyl in inerten Lösungsmitteln mit oder ohne Zusatz einer Base, z.B. Kaliumcarbonat, Natriumhydrid oder einem Alkalisalz von H-X-$C_1$-$C_4$-Alkyl, in einem inerten, bevorzugt dipolar aprotischen Lösungsmittel, wie z.B. Dimethylformamid oder Dimethylsulfoxid, bei -20° bis 150°C; oder

14.2 Verbindungen der Formel $Iu_2$ werden hergestellt durch Umsetzung von Verbindungen der Formel Ic und Id mit einem Alkohol der Formel HX-$C_1$-$C_4$-Alkyl in Gegenwart eines sauren Katalysators, wie z.B. Schwefelsäure, p-Toluolsulfonsäure oder Oxalsäure, mit oder ohne Zusatz einer Lewis-Säure, z.B. $AlCl_3$ oder Bortrifluoridetherat, mit

azeotroper Destillation oder mittels eines Molekularsiebes in inerten Lösungsmitteln, wie z.B. Toluol, Dioxan oder Tetrahydrofuran, oder im Ueberschuss von Verbindungen der Formel HX-$C_1$-$C_4$-Alkyl bei 0° bis 180°C; oder

14.3 Verbindungen der Formel $Iu_3$, worin X Sauerstoff darstellt, werden hergestellt durch Umsetzung von Verbindungen der Formel IX oder deren Hydrohalogenide mit mindestens 2 Aequivalenten einer Verbindung der Formel HX-$C_1$-$C_4$-Alkyl mit oder ohne Zusatz einer Base, z.B. eines Alkalisalzes von Verbindungen der Formel HX-$C_1$-$C_4$-Alkyl, mit oder ohne inerte Lösungsmittel bei -30° bis 80°C; wobei X, $X_1$, $X_2$ und $X_3$ die unter Formel I angegebenen Bedeutungen besitzen; und

15. Verbindungen der Formeln $Iv_1$ und $Iv_2$

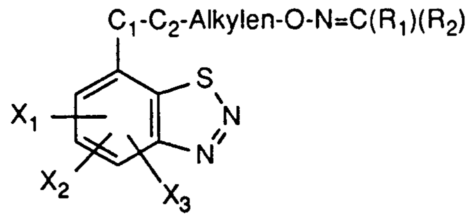

($Iv_1$)

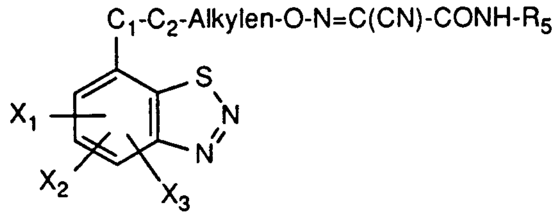

($Iv_2$)

werden hergestellt durch Umsetzung von Verbindungen der Formel Ie mit Verbindungen der Formeln M-O-N=C($R_1$)$R_2$ bzw. M-O-N=C(CN)CONH-$R_5$ in inerten Lösungsmitteln und falls M Wasserstoff bedeutet in Gegenwart einer Base, wie z.B. Alkalicarbonat, Natriumhydrid oder eines tert.Amins, z.B. Pyridin, bei -20° bis 140°C; wobei M Wasserstoff oder ein Alkaliatom bedeutet und $R_1$, $R_2$ und $R_5$ sowie $X_1$, $X_2$ und $X_3$ die unter Formel I angegebenen Bedeutungen besitzen; und

16. Verbindungen der Formel Iw

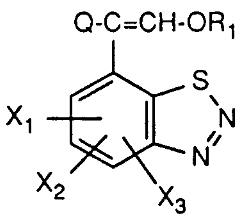

(Iw)

werden hergestellt:

16.1 durch Umsetzung von Verbindungen der Formel XVI

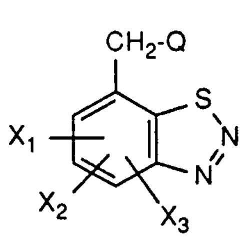

(XVI)

mit Ameisensäure-$C_1$-$C_4$-Alkylester oder Ameisensäure-Ortho-$C_1$-$C_4$-Alkylester in Gegenwart einer Base, wie z.B. Natriumalkoholat oder Natriumhydrid, in inerten Lösungsmitteln, wie z.B. Ether, Tetrahydrofuran oder Toluol, bei -10° bis 120°C und

16.2 anschliessend die erhaltenen Verbindungen der Formel Iw, worin $R_1$ Wasserstoff bedeutet, mit Verbindungen der Formel $R_1Hal$, worin $R_1$ $C_1$-$C_2$-Alkyl darstellt, unter Zusatz einer Base und eines dipolaren aprotischen Lösungsmittels, z.B. Dimethylformamid, bei 0° bis 80°C reagieren lässt; wobei Q, $R_1$, $X_1$, $X_2$ und $X_3$ die unter Formel I angegebenen Bedeutungen besitzen.

17. Verbindungen der Formel $Ix_1$ und $Ix_2$

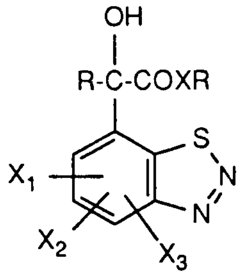

$(Ix_1)$

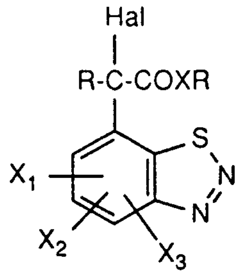

$(Ix_2)$

werden hergestellt durch hydrolytische Umsetzung von Verbindungen der Formeln $In_1$, $In_2$, $In_3$ und $In_4$ mit wässerigen Mineralsäuren oder durch basische Hydrolyse von Verbindungen der Formel Ih oder Ii mit Alkalihydroxid mit oder ohne Zusatz inerter Lösungsmittel, z.B. Tetrahydrofuran, bei -10° bis 180°C, bevorzugt 0° bis 100°C; wobei Hal Halogen bedeutet und X, R, $X_1$, $X_2$ und $X_3$ die unter Formel I angegebenen Bedeutungen besitzen. Hierbei werden die freien Säuren (R =Wasserstoff) erhalten. Diese können, falls gewünscht, mit einem Alkohol RXH in Gegenwart wasserabspaltender Katalysatoren, wie z.B. Bortrifluoridetherat, mit oder ohne Lösungsmittel bei 0° bis 160°C verestert werden; und

18. Verbindungen der Formel Iy

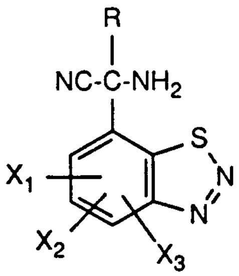

(Iy)

werden hergestellt:

18.1 durch Umsetzung von Verbindungen der Formel Ic oder Id mit Alkalicyanid und Ammoniak in Gegenwart von Ammoniumhalogenid mit oder ohne Zusatz von inerten Lösungsmitteln bei -20° bis 80°C oder

18.2 durch Umsetzung von Verbindungen der Formel $In_1$, $In_2$, $In_3$ oder $In_4$ mit Ammoniak in Gegenwart von Ammoniumhalogenid mit oder ohne Zusatz von inerten Lösungsmitteln bei -20 bis 80°C; wobei R, $X_1$, $X_2$ und $X_3$ die unter Formel I angegebenen Bedeutungen besitzen; und

19. Verbindungen der Formel Iz

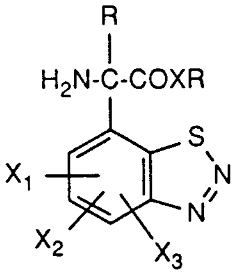

(Iz)

werden hergestellt durch Hydrolyse von Verbindungen der Formel Ig mit wässriger Mineralsäure, z.B. HCl, bei -20° bis 130°C; wobei R, X, $X_1$, $X_2$ und $X_3$ die unter Formel I angegebenen Bedeutungen besitzen.

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mikroorganismen mittels Blattapplikation oder Bodenapplikation als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der besonderen Wirkungsweise der erfindungsgemässen Verbindungen der Formel I, nämlich einerseits der Möglichkeit einer direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I eigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. Hemileia, Rhizocotonia, Puccinia); Ascomyceten (z.B. Venturia, Podosphaera, Erysiphe, Monilinia, Uncinula).

Darüberhinaus können die Wirkstoffe besonders vorteilhaft gegen folgende Schadorganismen eingesetzt werden:

Pilze, wie z.B. Oomyceten (z.B. Plasmopara viticola, Phytophthora infestans, Peronospora tabacina, Pseudoperonospora, Bremia letucae), Fungi imperfecti (z.B. Colletotrichum lagenarium, Pyricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen unterschiedlicher Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen-und Naturkautschukgewächse sowie Zierpflanzen (Blumen, Sträucher, Laubbäume und Nadelbäume wie Koniferen). Diese Aufzählung stellt eine Limitierung dar.

Als besonders geeignete Zielkulturen für die Anwendung des erfindungsgemässen Verfahrens sind folgende Pflanzen anzusehen: Gurke, Tabak, Reben, Reis, Pfeffer, Kartoffeln, Tomaten, Weizen, Gerste, Birnen und Aepfel.

Die im Rahmen der Erfindung zur Anwendung gelangenden mikrobiziden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusamrnensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aberauch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstofies der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indemman den Standort der Pflanze mit einer flüssigen Zubereitung tränkt oder die Substanzen in fester Form in den Boden einbringt z.B. in Form von Granulat. Die Verbindungen der Formel I können aber auch auf Samenkörller aufgebracht werden (Coating), indem man die Körnerentweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel 1 werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandnlengen liegen im allgemeinen bei 50 g bis 5 kg Aktivsubstanz (AS)je ha: bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel-oder Stearinsäure oder von natürlichen Fettsäuregemischen, die z.B. aus Kokosnuss-oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder-sulfate liegen in der Regel als Alkali-, Erdalkali- oder gegebenenfalls substituierte Ammoniumsalze vor und weisen einen Alkylrest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%. insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

1. Herstellungsbeispiele

Beispiel 1.1: Herstellung von 7-Methylsulfonyloxymethylbenz-1,2,3-thiadiazol (Verb.Nr. 1.20)

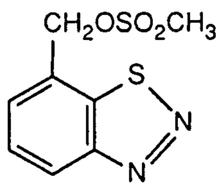

Zu einer Lösung von 3,32 g 7-Hydroxymethyl-benz-1.2.3-thiadiazol, 3,1 ml Triethylamin und 0,2 g 4-Dimethylaminopyridin in 50 ml Methylenchlorid wird unter Kühlung bei - 5° bis 0°C eine Lösung von 2,23 g Methansulfonylchlorid in 15 ml Dichlormethan zugetropft und 2 Std. bei Raumtemperatur gerührt. Dann wird das Reaktionsgemisch mit Eiswasser versetzt und mehrmals mit Dichlormethan extrahiert. Die vereinigten Extrakte werden mit Wasser gewaschen, getrocknet und eingedampft. Chromatographische Reinigung an Kieselgel ($CH_2Cl_2$) liefert die Titelverbindung als viskoses Harz.

Beispiel 1.2: Herstellung von Benz-1,2,3-thiadiazol-7-carbaldehyd (Verb. Nr. 1.4)

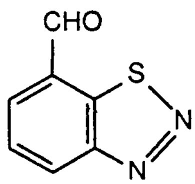

a) 99 g Benz-1,2,3-thiadiazol-7-carbonsäurechlorid werden in 1,2 l Essigsäureethylester gelöst und bei 0-5°C mit einer Lösung von 58,7 g 2,6-Lutidin in 200 ml Tetrahydrofuran sowie 33 g Palladium/Kohle-Katalysator (5% Pd) versetzt und bei Niederdruck ($10^4$ Pa) unter weiterem Zusatz von total 57 g Palladiuni/Kohle-Katalysator und 500 ml Tetrahydrofuran in 5 Portionen hydriert. Anschliessend wird vom Katalysator abfiltriert, mit Tetrahydrofuran nachgewaschen und das Filtrat eingedampft. Der Rückstand wird an Kieselgel ($CH_2Cl_2$) gereinigt und liefert die Titelverbindung vom Smp. 134-136°C.

b) Zu einer Lösung von 16,6 g 7-Hydroxymethyl-benz-1,2,3-thiadiazol in 100 ml Chloroform werden unter Rühren bei Raumtemperatur 35 g Mangandioxid eingetragen, wobei sich die Innentemperatur für kurze Zeit auf 31°C erhöht. Nachdem wieder 25°C erreicht ist, wird aufgeheizt und über Nacht unter Riickfluss gekocht. Anschliessend werden nochmals 5 g Mangandioxid zugesetzt und bis zur völligen Umsetzung wird weiter erhitzt. Nach Filtration des heissen Gemisches über Hyflo wird eingedampft. Der Rückstand wird in wenig Hexan digeriert und dann filtriert, wobei 15 g (91,4 %) der Titelverbindung vom Smp. 134-136°C erhalten werden.

Beispiel 1.3: Herstellung von 7-Trichlormethyl-benz-1,2,3-thiadiazol (Verb. Nr. 1.2)

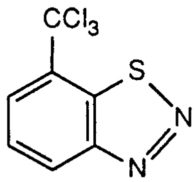

Unter Stickstoffatmosphäre und Rühren werden zu 9,0 g 7-Carboxy-benz-1,2,3-thiadiazol und 10,7 g Benzolphosphonsäuredichlorid portionenweise 26 g Phosphorpentachlorid eingetraten. Dann wird das Gemisch (in fester Form vorliegend) auf 160°C aufgeheizt, wobei bei ca. 100°C Schmelze eintritt. Nach 16 Std. Rühren bei 160°C wird abgekühlt, vorsichtig auf Eis gegeben und mit festem Natriumcarbonat alkalisch gestellt. Dann wird mehrmals mit Essigsäureethylester extrahiert, die Extrakte mit Wasser gewaschen, über Hyflo filtriert und eingedampft. Reinigung an Kieselgel (Hexan mit steigendem Zusatz an Essigsäurethylester bis zu einem Verhältnis von 8:2) liefert weisse Kristalle vom Smp. 67-69°C.

Beispiel 1.4: Herstellung von 7-Dichlormethyl-benz-1,2,3-thiadiazol (Verb. Nr. 1.1)

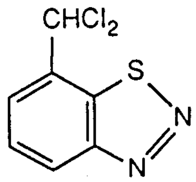

Zu vorgelegten 8,7 ml Thionylchlorid werden unter Kühlen 0,5 ml Triethylamin gegeben und dann bei 15-20°C portionenweise 6,6 g Benz-1,2,3-thiadiazol-7-carbaldehyd eingetragen und die Mischung anschliessend während 1 Std. auf 45°C und dann weiter während 4 Std. auf 95°C erwärmt, wobei leichter Gasabgang zu beobachten ist. Die entstandene klare Lösung wird anschliessend eingedampft, in Methylenchlorid aufgenommen und mit Eiswasser gewaschen. Die organische Phase wird über Natriumsulfat getrocknet und eingedampft. Der Rückstand wird aus Essigsäureethylester-Hexan umkristallisiert, wobei die Titelverbindung vom Smp. 131-133°C resultiert.

Beispiel 1.5: Herstellung von 7-Chlormethyl-benz-1,2,3-thiadiazol (Zwischenprodukt)

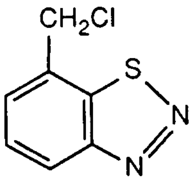

199,2 g der Verbindung von Beispiel 1.15 werden in 150 ml Methylenchlorid und 150 ml Pyridin bei 5°C gelöst, vorgelegt und unter Kühlen bei 5-10°C innerhalb 3/4 Std. mit einer Lösung von 120 ml Thionylchlorid in 200 ml Methylenchlorid versetzt. Dann wird über Nachtbei bei Raumtemperatur weitergerührt, die Suspension auf Eiswasser gegossen und mit Methylenchlorid extrahiert. Die Extrakte werden mit Eiswasser gewaschen, über Natriumsulfat getocknet, filtriert und eingedampft. Die so erhaltene Titelverbindung schmilzt bei 78-80°C.

Beispiel 1.6: Herstellung von 7-Acetyl-benz-1,2,3-thiadiazol (Verb. Nr. 4.2)

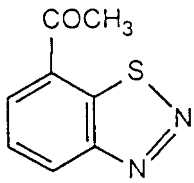

Unter Rühren werden zu 0,59 g Magnesiumspänen in 3 ml Diethylether nach Aktivierung mit 1 Tropfen Brom eine Lösung von 1,5 ml Methyljodid in 5,5 ml Diethylether zugetropft. Nach Abklingen der exothermen Reaktion wird aufgeheizt und noch 1,5 Std. auf 55°C Badtemperatur gehalten. Die erhaltene Lösung wird unter Stickstoffatmosphäre und Rühren bei -10°C zur vorgelegten Lösung von 4,9 g N-Methyl-N-methoxy-benz-1,2,3-thiadiazol-7-carbonsäureamid in 150 ml abs. Tetrahydrofuran getropft. Nachdem während 1 Std. bei -10°C weitergerührt worden ist, wird aufgeheizt und bei 75°C Badtemperatur während 6 Std. unter Rückfluss gekocht. Dann wird das Reaktionsgemisch abgekühlt, auf Eiswasser gegossen, mit 2N Salzsäure angesäuert und mehrmals mit Essigsäureethylester extrahiert. Die Extrakte werden mit Wasser gewaschen, über Natriumsulfat getrocknet und eingedampft. Der Rückstand wird an Kieselgel ($CHCl_3$) gereinigt, wobei die Titelverbindung vom Smp. 126-127°C erhalten wird.

Beispiel 1.7: Herstellung von 7-Carboxymethyl-benz-1,2,3-thiadiazol (Verb.Nr. 1.39)

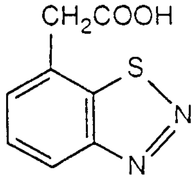

1,9 g 7-Cyanobenz-1,2,3-thiadiazol werden über Nacht in 80 ml konz. Salzsäure erhitzt. Dann wird eingedampft, mit wenig kaltem Wasser versetzt und der entstandene Feststoff abfiltriert und mit Wasser gewaschen. Der Rückstand wird getrocknet, wobei 1,7 g der Titelverbindung vom Smp. 148-150°C resultiert.

Beispiel 1.8: Herstellung von 3-[Benzo-1,2,3-thiadiazol-7-yl]-acrylsäure (Verb.Nr. 1.98)

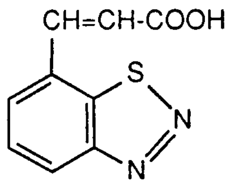

Ein Gemisch von 1,96g Benz-1,2,3-thiadiazol-7-carbaldhyd, 1,24 g Malonsäure und 6 ml Pyridin werden während 26 Std. auf 120°C erhitzt, wobei nach 4 und 7 Std. 2 weitere Zugaben von je 0,6 g Malonsäure erfolgen. Dann wird abgekühlt, auf Eiswasser gegossen und mit konz. Salzsäure angesäuert. Vom ausgefallenen Niederschlag wird abfiltriert, dieser in verdünnter Natronlauge gelöst und dreimal mit Methylenchlorid gewaschen und anschliessend die Wasserphase wieder mit Salzsäure angesäuert. Der erhaltene Niederschlag wird abfiltriert, mit Wasser gewaschen und unter Vakuum getrocknet, wobei 11 g der Titelverbindung vom Smp. 199-202°C resultieren.

Beispiel 1.9: Herstellung von 7-Cyanomethyl-benz-1,2,3-thiadiazol (Verb.Nr. 1.38)

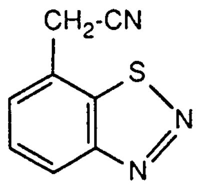

18,4 g 7-Chlormethyl-benz-1,2,3-thiadiazol werden in 50 ml Chloroform gelöst, mit 0,5 g Tetrabutylammoniumjodid versetzt, auf 50-55°C erwärmt und unter Rühren innerhalb 0,5 Std. tropfenweise mit einer Lösung von 5,6 g Natriumcyanid in 20 ml Wasser versetzt. Ueber Nacht wird bei der gleichen Temperatur weiter erwärmt, anderntags abgekühlt, mit Wasser versetzt und mehrmals mit Dichlormethan extrahiert. Die Extrakte werden mit Wasser gewaschen, über Natriumsulfat getrocknet, über Hyflo filtriert und das Filtrat eingedampft. Der Rückstand wird an Kieselgel (Hexan/Essigsäureethylester) gereinigt, wobei die Titelverbindung vom Smp. 78-80°C resultiert.

Beispiel 1.10: Herstellung von 3-[Benz-1,2,3-thiadiazol]-3-oxo-2,2-diethoxypropionsäureethylester (Verb. Nr. 1.64)

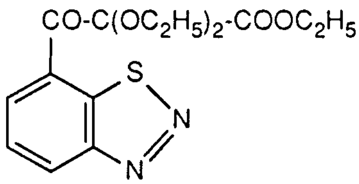

71,5 g Benz-1,2,3-thiadiazol-7-carbonsäurechlorid und 88,1 g Tetraethoxyethylen werden in 180 ml Toluol

während 17 Std. unter Rückfluss gekocht. Danach wird das Gemisch abgekühlt, mit Natriumbicarbonat-Lösung und Wasser gewaschen, über Natriumsulfat getrocknet und eingedampft. Der feste Rückstand wird in heissem Tetrahydrofuran gelöst und unter Rühren mit Hexan ausgefällt. Es resultieren 61,5 g der Titelverbindung vom Smp. 123-125°C.

Beispiel 1.11: Herstellung von 7-Trifluormethyl-benz-1,2,3-thiadiazol

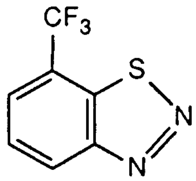

38,8 g 3,5-Diamino-3-benzylthiobenzotrifluorid in 100 ml Dioxan werden unter kräftigem Rühren bei 0°C unter Kühlen zu 195 ml 5N Salzsäure getropft. Anschliessend wird auf -15°C abgekühlt und unterhalb des Flüssigkeits-Niveaus tropfenweise mit 18,6 g Natriumnitrit in 150 ml Wasser versetzt. Anschliessend wird die rote Lösung während 6 Std. bei -5°C gerührt und schliesslich unter Rühren auf -10°C abgekühlte unterphosphorige Säure (71 ml) gegossen. Ueber Nacht wird unter Abkühlung auf Raumtemperatur weiter gerührt, anderntags mehrmals mit Methylenchlorid extrahiert, die Extrakte mit Wasser gewaschen, über Natriumsulfat getrocknet und dann eingedampft. Der ölige Rückstand wird durch mehrmalige Destillation im Kugelrohr bei 80°C/7,8 Pa vom Benzylchlorid befreit.

Beispiel 1.12: Herstellung von 3,5-Diamino-2-benzylthiobenzotrifluorid (Zwischenprodukt)

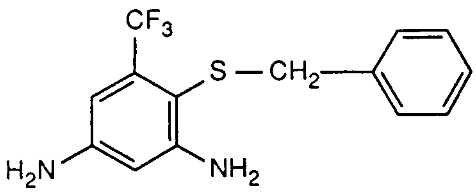

3 g 2-Benzylthio-3,5-dinitrobenzotrifluorid werden in einer Hydrierapparatur aus Glas in 30 ml Tetrahyrofuran mit total 3 g Raney-Nickel innerhalb 22 Std. bei Zimmertemperatur hydriert. Zur Aufarbeitung wird vom Katalysator abfiltriert, mit Tetrahydrofuran nachgewaschen, das Filtrat eingedampft und der Rückstand als Rohprodukt (bräunliche Flüssigkeit) direkt weiterverarbeitet.

Beispiel 1.13: Herstellung von 2-Benzylthio-3,5-dinitrobenzotrifluorid (Zwischenprodukt)

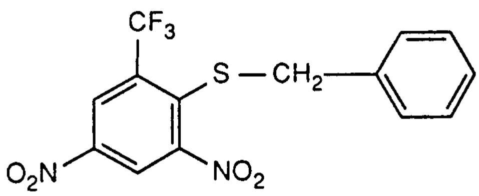

67,6 g 2-Chlor-3,5-dinitrobenzotrifluorid werden in 150 ml N,N-Dimethylformamid (DMF) gelöst, mit 35,9 g festem Kaliumcarbonat versetzt und bei 0°C unter Rühren tropfenweise mit 31 g Benzylmercaptan in 200 ml DMF versetzt und anschliessend weitere 16 Std. unter Abkühlung auf Raumtemperatur gerührt. Dann wird das Reaktionsgemisch mit Eiswasser versetzt und mehrfach mit Methylenchlorid extrahiert. Die Extrakte werden mit Wasser gewaschen, getrocknet und eingedampft. Der Rückstand wird mit Hexan unter Erwärmen digeriert und filtriert. Es werden gelbe Kristalle vom Smp. 93-95°C erhalten.

Beispiel 1.14: Herstellung von Benzo-1,2,3-thiadiazol-7-ethylester-imid-hydrochlorid (Zwischenprodukt)

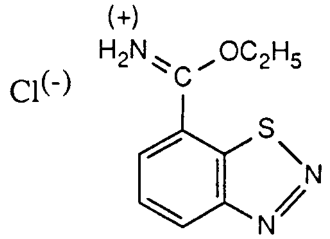

7,2 g 7-Cyano-benzo-1,2,3-thiadiazol werden in 60 ml abs. Ethanol und 20 ml Tetrahydrofuran bei 45°C gelöst, auf 0°C abgekühlt und die Lösung unter Feuchtigkeits-Ausschluss bei 0°C bis 15°C mit gasförmiger Salzsäure innerhalb 1 Std. gesättigt. Dann wird das Reaktionsgemisch während 24 Std. im Kühlschrank aufbewahrt, mit 70 ml abs. Diethylether versetzt und weitere 4 Tage im Kühlschrank stehen gelassen. Dann wird abfiltriert, mit Diethylether gewaschen und getrocknet. Es werden 9,4 g der Verbindung 5.2 als beige Kristalle vom Smp. 270-272°C erhalten.

Beispiel 1.15: Herstellung von 7-Hydroxymethl-benzo-1,2,3-thiadiazol (Zwischenprodukt)

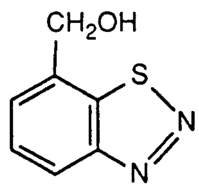

Zu einer Suspension von 54 g Carboxybenz-1,2,3-thiadiazol in 420 ml Tetrahydrofuran werden unter Stickstoffatmosphäre und Rühren bei Raumtemperatur 110 ml Triethylborat zugetropft, 1 Std. weitergerührt und anschliessend unter leichtem Kühlen tropfenweise mit 45,6 ml Borandimethylsulfid-Komplex in 60 ml Tetrahydrofuran versetzt, wobei heftige Gasentwicklung beobachtet wird. Nach Rühren über Nacht und Stehenlassen bei Raumtemperatur wird wieder auf 5-10°C abgekühlt und unter Rühren und starkem Kühlen tropfenweise mit 200 ml Methanol versetzt, wobei wiederum starke Gasentwicklung auftritt. Anschliessend wird unter Vakuum eingedampft, nochmals 300 ml Methanol zugegeben und wieder eingedampft. Der Rückstand wird an Kieselgel (Lösungsmittel: Essigsäureethylester/Hexan) gereinigt und das erhaltene Produkt aus Essigsäureethylester/Hexan umkristallisiert. Die erhaltene Titelverbindung schmilzt bei 79-81°C.

Beispiel 1.16: Herstellung von 6-Chlor-7-hydroxymethyl-benzo-1,2,3-thiadiazol (Zwischenprodukt)

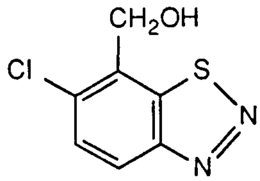

Zu einer Suspension von 4,5 g Natriumborhydrid in 30 ml Wasser und 70 ml Tetrahydrofuran werden unter Rühren und Erwärmen auf 40-50°C innerhalb einer halben Stunde eine Lösung von 1,9 g 6-Chlor-7-carbomethoxybenzo-1,2,3-thiadiazol in 30 ml Tetrahydrofuran (warm gelöst) zugetropft. Bis zur völligen Umsetzung wird weiter erwärmt, mit einem $CO_2$-Kühlbad auf -20° bis -30°C abgekühlt und tropfenweise mit 15 ml Aceton versetzt, wobei starke Gasentwicklung beobachtet wird. Die Reaktionsmischung wird anschliessend mit 15%iger Salzsäure unter weiterer kräftiger Kühlung bei -20° bis -10°C auf pH 3 angesäuert (Gasentwicklung) und über Nacht weitergerührt. Dann wird der grösste Teil des Tetrahydrofurans am Rotationsverdampfer abgezogen und der Rückstand mit Essigsäureethylester extrahiert. Die Extrakte werden mit Wasser gewaschen, über Natriumsulfat getrocknet und eingedampft. Der Rückstand liefert die Titelverbindung vom Smp. 138-140°C.

Beispiel 1.17: Herstellung von 2-(Benz-1,2,3-thiadiazolyl)-2-hydroxy-iminoacetonitril (Verb. Nr. 1.127)

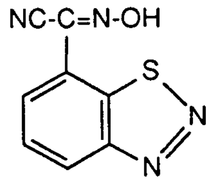

Eine Suspension von 0,36 g Kalium-tert.-butylat in 5 ml abs. Diethylether wird bei - 5°C innerhalb 10 Min. tropfenweise miteiner Lösung von 0.5 g 7-Cyanomethyl-benz-1,2,3-thiadiazol und 0,43 ml Isoamylnitrit in 10 ml abs. Tetrahydrofuran versetzt und bei Raumtemperatur während total 48 Std. gerührt, wobei in Abständen von 16, weiteren 16 und 8 Std. noch dreimal je 0,43 ml Isoamylnitrit zugegeben werden. Dann wird mit Eiswasser verdünnt, mit 1N Salzsäure angesäuert und mit Essigsäureethylester extrahiert. Die Extrakte werden mit Wasser gewaschen, über Natriumsulfat getrocknet und eingedampft. Chromatographie des Rückstandes an Kieselgel (Essigsäureethylester/Hexan 1:2) liefert die Titelverbindung vom Smp. 249-253°C.

Beispiel 1.18: Herstellung von 7-(1,1,1-Trichlor-2-hydroxy-ethyl)-benz-1,2,3-thiadiazol (Verb. Nr. 1.108)

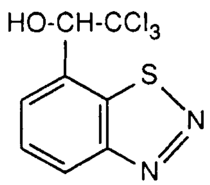

Eine Mischung von 5,8 g Trichloressigsäure und 2,6 g 7-Formyl-benz-1,2,3-thiadiazol in 20 ml Hexamethylphosphorsäuretriamid wird während 4 Std. auf 70°C erwärmt, wobei unter $CO_2$-Abgang rasch Lösung eintritt. Dann wird abgekühlt, auf Eiswasser gegossen und mit Essigsäureethylester extrahiert. Die Extrakte werden mit Wasser gewaschen, getrocknet und eingedampft. Der Rückstand wird aus Essigsäureethylester/Hexan umkristallisiert, wobei die Titelverbindung vom Smp. 163-165°C resultiert.

Beispiel 1.19: Herstellung von 7-Aminomethyl-benz-1,2,3-thiadiazol (Verb. Nr. 1.110)

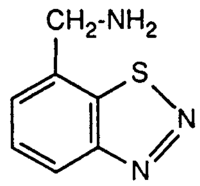

Eine Lösung von 14,5 g 7-Cyano-benz-1,2,3-thiadiazol in 150 ml Methanol wird in einem Hydriergefäss mit 4,4 g Raney-Nickel versetzt und mit 15 g flüssigem Stickstoff versetzt. Anschliessend wird Wasserstoff aufgepresst, aufgeheizt und bei 60°C und konstantem Wasserstoff-Druck von $10^7$ Pa bis zur Beendigung der Wasserstoff-Aufnahme hydriert. Dann wird abgekühlt, vom Katalysator abfiltriert, das Filtrat eingedampft und an Kieselgel (Hexan/Essigsäureethylester 2:1) chromatographiert, wobei die Titelverbindung vom Smp. 136-139°C resultiert.

Beispiel 1.20: Herstellung von 7-Trichloracetyl-benz-1,2,3-thiadiazol (Verb. Nr. 1.107)

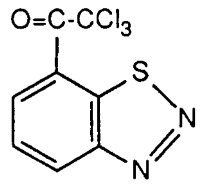

Eine Lösung von 2,8 g 7-(1,1,1-Trichlor-2-hydroxy-ethyl)-benz-1,2,3-thiadiazol in 30 ml Chloroform wird mit 5 g Mangandioxid versetzt und unter Rühren während 16 Std. unter Rückfluss gekocht. In Abständen von 16 Std. resp. 8 Std. wird nochmals 5 resp. 3 g Mangandioxid zugegeben. Nach total 40 Std. wird abgekühlt, über Hyflo zweimal filtriert, das Filtrat eingedampft und an Kieselgel (Hexan/Essigsäureethylester 7:3) gereinigt, wobei die Titelverbindung mit einem Brechungsindex $n_D^{26}$ = 1,6178 erhalten wird.

Beispiel 1.21: Herstellung von 7-Acetylthiomethyl-benz-1,2,3-thiadiazol (Verb. 1.113)

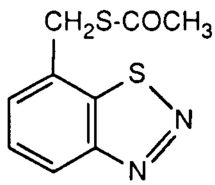

Eine bei 20°C gerührte Lösung von 2,2 g Kaliumthioacetat in 20 ml abs. Dimethylsulfoxid wird unter Rühren tropfenweise miteiner Lösung von 2,8 g 7-Chlormethyl-benz-1,2,3-thiadiazol in 10 ml Dimethylsulfoxid versetzt, wobei die Innentemperatur auf 37°C ansteigt. Anschliessend wird über Nacht bei Raumtemperatur gerührt, anderntags auf Eiswasser gegossen und mit Essigsäureethylester extrahiert. Die Extrakte werden mit Wasser fünfmal gewaschen, über Natriumsulfat getrocknet und eingedampft. Derverbleibende Rückstand wird am Vakuum destilliert, wobei ein Oel mit einem Brechungsindex $n_D^{27}$ = 1,6478 resultiert.

Beispiel 1.22: Herstellung von 7-Dimethoxymethyl-benz-1,2,3-thiadiazol (Verb. Nr. 1.12)

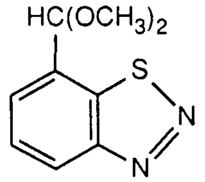

Eine Lösung von 3,3 g 7-Formyl-benz-1,2,3-thiadiazol in 50 ml Methanol und 0,2 g p-Toluolsulfonsäure wird in einermit einem Molekularsieb $A_4$ beschichteten SoxhletApparatur während 6 Std. unter Rückfluss gekocht. Dann wird abgekühlt, mit festem Kaliumcarbonat basisch gestellt, über Hyflo filtriert und eingedampft. Der Rückstand wird an Kieselgel mit Hexan/Essigsäureethylester (1:1) gereinigt, wobei die Titelverbindung mit einem Brechungsindex $n_D^{21}$ = 1,5750 resultiert.

Beispiel 1.23: Herstellung von Benz-1,2,3-thiadiazol-7-N-Methoxyhydroxamsäure (Verb. Nr. 1.112)

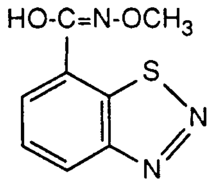

Eine bei -5°C gerührte Suspension von 2,4 g O-Methyl-hydroxylamin-Hydrochlorid, 4,5 g Kaliumcarbonat und 20 ml Dichlormethan wird unter Kühlen und Rühren tropfenweise mit einer Lösung von 4,96 g Benz-1,2,3-thiadiazol-7-carbonsäurechlorid in 25 ml Dichlormethan versetzt. Ueber Nacht wird bei Raumtemperatur gerührt, filtriert, das Filtrat eingedampft und der Rückstand an Kieselgel (Essigsäureethylester/Tetrahydrofuran 1:1) gewaschen. Mai, erhält die Titelverbindung vom Smp. 188-190°C.

Beispiel 1.24: Herstellung von 7-Methoxymethyl-benz-1,2,3-thiadiazol (Verb. Nr. 1.7)

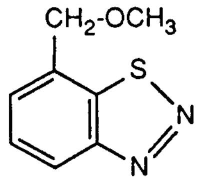

Eine Lösung von 4,15 g 7-Hydroxymethylbenz-1,2,3-thiadiazol in 15 ml Tetrahydrofuran wird unter Stickstoffatmosphäre und Rühren bei 0-5°C zu vorgelegten 1,4 g Natriumhydrid (50 %ige Oeldispersion) in 15 ml abs. Dimethylsulfoxid getropft, wobei sich Wasserstoff entwickelt. Nach 20 Min. Rühren bei 10°C wird wieder auf 0-5°C abgekühlt und eine Lösung von 4 g Methyljodid in 15 ml Tetrahydrofuran zugetropft. Es wird noch 1 Std. bei Raumtemperatur gerührt, abgekühlt, mit Eiswasser versetzt, mit verd. Salzsäure neutralisiert und mit Essigsäureethylester extrahiert. Die Extrakte werden mit Wasser gewaschen, über Natriumsulfat getrocknet, eingedampft und am Hochvakuum getrocknet, wobei die Titelverbindung als gelbes Oel, $n_D^{27}$ = 1,5912 resultiert.

Beispiel 1.25: Herstellung von 7-Oximino-benz-1,2,3-thiadiazol (Verb. Nr. 1.65)

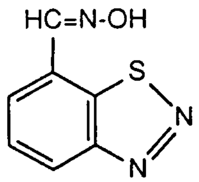

Eine Lösung von 4,9 g 7-Formyl-benz-1,2,3-thiadiazol in 70 ml abs. Methanol wird mit 2,1 g Hydroxylamin-Hydrochlorid und 2,4 g Natriumacetat versetzt und während 6 Std. bei 70°C gehalten. Dann wird das Lösungsmittel am Vakuum grösstenteils eingeengt, der Rückstand mit Eiswasser versetzt, der Niederschlag abfiltriert, mit Wasser gewaschen und getrocknet, wobei die Titelverbindung vom Smp. 230-231°C (Zers.) resultiert.

Beispiel 1.26: Herstellung von 7-Methoxyinlinobenz-1,2,3-thiadiazol (Verb. Nr. 1.66)

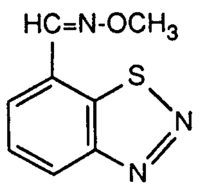

Eine Suspension von 0,92 g Natriumhydrid (50 % Oelsdispersion) in 10 ml Dimethylsulfoxid und 10 ml Tetrahydrofuran wird unter Stickstoffatmosphäre und Rühren bei 0-10°C tropfenweise mit einer Lösung von 3,0 g 7-Oximinobenz-1,2,3-thiadiazol in 10 ml Tetrahydrofuran versetzt und nach 0,5 Std. Rühren bei 15-20°C mit 2,7 g Methyljodid in 10 ml Tetrahydrofuran versetzt. Nach Rühren während 5 Std. bei Raumtemperatur wird mit Eiswasser versetzt, mit

Methylenchlorid extrahiert, die Extrakte mit Wasser gewaschen und getrocknet. Der nach Eindampfen verbleibende Rückstand wird mit Hexan digeriert, wobei hellgelbe Kristalle vom Smp. 121-123°C resultieren.

Beispiel 1.27: Herstellung von 7-(3-Trifluormethylphenyl-imino)-benz-1,2,3-thiadiazol (Verb. Nr. 1.79)

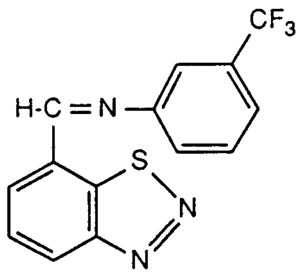

Eine Lösung von 2,46 g 7-Formyl-benz-1,2,3-thiadiazol und 2,41 g 3-Aminobenzotrifluorid wird mit einer Spatelspitze p-Toluolsulfonsäure und ca. 5 g Molekularsieb $A_4$ in 50 ml Toluol während 16 Std. bei Raumtemperatur gerührt. Dann wird filtriert, das Filtrat eingedampft, mit Hexan gewaschen und getrocknet, wobei die Titelverbindung vom Smp. 120-122°C resultiert.

Beispiel 1.28: Herstellung des 2,4,6,-Trichlorphenylhydrazons von Benz-1,2,3-thiadiazol-7-carbaldehyd (Verb. Nr. 1.89)

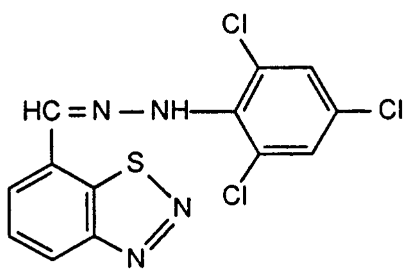

2,11 g 2,4,6-Trichlorphenylhydrazin in 30 ml Methanol und 20 ml Tetrahydrofuran, werden heiss gelöst, auf 40°C abgekühlt und unter Rühren mit einer Lösung von 2,46 g Benz-1,2,3-thiadiazol-7-carbaldehyd in 10 ml Methanol gegossen. Nach 4 Std. Rühren bei Raumtemperatur werden 8 ml Eisessig zugegeben und bis zur vollständigen Umsetzung bei 40°C weiter gerührt. Dann wird filtriert, mit Methanol gewaschen und getrocknet, wobei die Titelverbindung als beiger Feststoff (Smp. >250°C) resultiert.

Beispiel 1.29: Benz-1,2,3-thiadiazol-7-(N-hydroxycarboximidamid) (Verbindung 1.106)

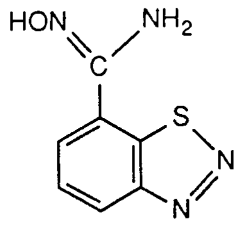

Eine vorgelegte Lösung von 5,0 g 7-Cyanobenz-1,2,3-thiadiazol und 2,5 g Hydroxylamin-Hydrochlorid in 34 ml Ethanol und 16 ml Wasser wurden portionenweise mit 6,4 g Kaliumcarbonat versetzt, wobei die Innentemperatur von 24° auf 36°C anstieg und leichte $CO_2$-Entwicklung zu beobachten war. Nach 1 1/2 Std. Rühren bei Raumtemperatur wurde noch 3 Std. unter Rückfluss gekocht, die entstandene Suspension mit Eiswasser versetzt, abfiltriert und mit Wasser und wenig Diethylether gewaschen. Der Niederschlag entsprach der Titelverbindung mit einem Smp. von 209-211°C.

Beispiel 1.30: Herstellung von 7-Bromacetyl-benz-1,2,3-thiadiazol (Verbindung 1.125)

0,9 g 7-Acetyl-benz-1,2,3-thiadiazol wurden in 20 ml Chloroform gelöst und bei Raumtemperatur tropfenweise mit 0,88 g Brom versetzt. Anschliessend wurde 2 Std. bei der gleichen Temperatur wobei sich ein gelberNiederschlag bildete. Dieser wurde abfiltriert, mit Hexan gewaschen und getrocknet. Die aufdiese Weise erhaltene Titelverbindung schmilzt bei 155-157°C.

Beispiel 1.31: Herstellung von N,N'-Diphenyl-C-[benz-1,2,3-thiadiazol-7'-yl]-formazan (Verbindung Nr. 4.1)

1,65 g Anilin werden in Gegenwart von 4 ml Chlorwasserstoffsäure (30%ig) und 1,8g Eis mit einer Lösung von 1,25 g Natriumnitrit in 2,2 ml Wasser bei 0°C diazotiert. Die erhaltene Lösung des Diazonium-Salzes wird unter Rühren und Kühlen bei - 5° bis 0°C zu der vorgelegten Lösung von 4,6g Benz-1,2,3-thiadiazol-7-carbaldehyd-phenylhydrazon in 60 ml Pyridin und 90 ml Ethanol zugetropft und das Reaktionsgemisch 18 Std. bei Zimmertemperatur weitergerührt. Anschliessend wird am Vakuum eingeengt und mit Ethylacetat extrahiert. Die Extrakte werden mit Wasser gewaschen, getrocknet und eingedampft. Der Rückstand wird an Kieselgel (Ethylacetat/Hexan 9:1) gereinigt, wobei die Titelverbindung, Smp. 185-187°C, resultiert.

Tabelle 1

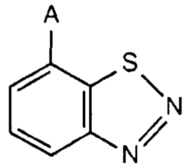

| Verb. Nr. | A | Physik. Daten |
|---|---|---|
| 1.1 | $CHCl_2$ | Smp. 131-133°C |
| 1.2 | $CCl_3$ | Smp. 67-69°C |
| 1.3 | $CH(Cl)CH_3$ | |
| 1.4 | $CBr_3$ | |
| 1.5 | $H_3C\text{-}C(OCH_3)_2$ | |
| 1.6 | $CH_2OCOCH_3$ | Smp. 52-54°C |
| 1.7 | $CH_2OCH_3$ | $n_D^{27} = 1.5912$ |
| 1.8 | $CH_2O$-CO-Cyclopropyl | |
| 1.9 | $CH(OH)CH_3$ | |
| 1.10 | $C(OCH_3)_3$ | |
| 1.11 | $C(OC_2H_5)_3$ | |
| 1.12 | $CH(OCH_3)_2$ | $n_D^{21} = 1.5750$ |
| 1.13 | $CH(OC_2H_5)$ | |
| 1.14 | $CH[OCH(CH_3)_2]_2$ | |
| 1.15 | $CH(OC_4H_9\text{-n})_2$ | |
| 1.16 | $CH(SCH_3)CH_3$ | |
| 1.17 | $CF_3$ | Sdp. 80°C/7,8 Pa |
| 1.18 | $CH_2SCH_3$ | $n_D^{27} = 1.6311$ |
| 1.19 | $CH(S\text{-iso}C_3H_7)_2$ | |
| 1.20 | $CH_2O\text{-}SO_2CH_3$ | Harz |
| 1.21 | $CH_2O\text{-}SO_2\text{-}C_2H_5$ | |
| 1.22 | $CH_2O\text{-}SO_2$-Cyclohexyl | |
| 1.23 | $CH_2O$-CO-Cyclopropyl | |
| 1.24 | $CH(CH_3)O\text{-}COCH_3$ | |
| 1.25 | $CH_2OCO\text{-}CH_2CH{=}CH_2$ | |
| 1.26 | $CH_2OCO\text{-}CH_2\text{-}C{\equiv}CH$ | |

| Verb. Nr. | A | Physik. Daten |
|---|---|---|
| 1.27 | $CH_2OCO\text{-}CH_2CH{=}CH\text{-}CH_3$ | |
| 1.28 | $CH_2OCO\text{-}CH_2\text{-}CH{=}CH\text{-}C_2H_5$ | |
| 1.29 | $CH_2OCO\text{-}C_6H_{13}\text{-n}$ | |
| 1.30 | $CH(CH_3)OCO$-Cyclohexyl | |
| 1.31 | $CH_2OCO$-Phenyl | Smp. 73-74°C |
| 1.32 | $CH_2OCO$-2-Hydroxyphenyl | |
| 1.33 | $CH_2OCO\text{-}C(Cl){=}C(Cl)_2$ | Smp. 123-124°C |
| 1.34 | $CH_2O$-Tosyl | |
| 1.35 | $CH_2O\text{-}SO_2$-4-Bromphenyl | |
| 1.36 | $CH(CH_3)O\text{-}SO_2CH_3$ | |
| 1.37 | $CH_2O\text{-}SO_2C_6H_{13}\text{-n}$ | |
| 1.38 | $CH_2CN$ | Smp. 78-80°C |
| 1.39 | $CH_2COOH$ | Smp. 148-150°C |
| 1.40 | $CH_2CONH_2$ | |
| 1.41 | $CH_2COOCH_3$ | |
| 1.42 | $CH_2COOC_4H_9$-tert. | |
| 1.43 | $CH_2CO\text{-}SCH_3$ | |
| 1.44 | $CH_2CO$-S-Phenyl | |
| 1.45 | $CH_2COO$-Phenyl | |
| 1.46 | $CH_2COO$-2-Hydroxyphenyl | |
| 1.47 | $CH(CH_3)\text{-}COOCH_3$ | |
| 1.48 | $CH(OH)COOH$ | |
| 1.49 | $CH(OH)\text{-}COOCH_3$ | |
| 1.51 | $CH(CH_3)Br$ | |
| 1.52 | $CH(OH)CN$ | |
| 1.53 | $C(CH_3)(OH)CN$ | |
| 1.54 | $CH(OCH_3)CN$ | |
| 1.55 | $CH[OSi(CH_3)_3]CN$ | |
| 1.56 | $CH(NH_2)CN$ | |
| 1.57 | $CH(NH_2)COOCH_3$ | |
| 1.58 | $CH(NH_2)COOH$ | |
| 1.59 | $COCOOH$ | |

| Verb. Nr. | A | Physik. Daten |
|---|---|---|
| 1.60 | $COCOOCH_3$ | |
| 1.61 | $COCOOC_2H_5$ | |
| 1.62 | $COCOOC_5H_{11}$-n | |
| 1.63 | $COC(OCH_3)_2COOCH_3$ | |
| 1.64 | $COC(OC_2H_5)_2COOC_2H_5$ | Smp. 123-125°C |
| 1.65 | C≡CH | |
| 1.66 | $CH=NOCH_3$ | Smp. 121-123°C |
| 1.67 | $CH=NOC_2H_5$ | |
| 1.68 | $CH=NOC_4H_9$-i | |
| 1.69 | $C(CH_3)=NOH$ | |
| 1.70 | $C(CH_3)=NOCH_3$ | |
| 1.71 | CH=NH | |
| 1.72 | $CH=NCH_3$ | |
| 1.73 | CH=N-Phenyl | |
| 1.74 | CH=N-Cyclohexyl | |
| 1.75 | CH=N-Cyclopropyl | |
| 1.76 | CH=N-Allyl | |
| 1.77 | $C(CH_3)$=N(3,5-diCl-Phenyl) | |
| 1.78 | CH=N(3-$NO_2$-Phenyl) | |
| 1.79 | CH=N(3-$CF_3$-Phenyl) | Smp. 120-122°C |
| 1.80 | $CH_2$-O-N=$C(CH_3)_2$ | |
| 1.81 | $CH_2$-O-N=$C(CH_3)C_2H_5$ | |
| 1.82 | $CH_2ON=C(CN)CH_3$ | |
| 1.83 | $CH_2ON=C(CN)CONH_2$ | Smp. 157-159°C |
| 1.84 | $CH_2ON=C(CN)CONHCONHC_2H_5$ | |
| 1.85 | CH=N-$NH_2$ | Smp. 156-158°C |
| 1.86 | CH=N-NH-$C_2H_5$ | |
| 1.87 | CH=N-$N(CH_3)_2$ | |
| 1.88 | CH=N-NH-Phenyl | Smp. 167-168°C |
| 1.89 | CH=N-NH-2,4,6-TriCl-Phenyl | Smp. >250°C |
| 1.90 | CH=N-NH-2,6-Di-Cl-Phenyl | |
| 1.91 | CH=N-NH-Cyclohexyl | |
| 1.92 | CH=N-Benzyl | |

| Verb. Nr. | A | Physik. Daten |
|---|---|---|
| 1.93 | $C(CH_3)=N-NH-3-NO_2$-Phenyl | |
| 1.94 | COCN | |
| 1.95 | $CH(OCH_3)COOCH_3$ | |
| 1.96 | $CH(Cl)COOCH_3$ | |
| 1.97 | $CH(Cl)COOC_2H_5$ | |
| 1.98 | CH=CHCOOH | Smp. 199-202°C |
| 1.99 | CH=C(CN)COOH | |
| 1.100 | CH=CHCN | |
| 1.101 | $CH=C(CN)COOCH_3$ | Smp. 150-153°C |
| 1.102 | $CH=C(COOC_2H_5)_2$ | |
| 1.103 | $CH_2-CH_2COOH$ | |
| 1.104 | $CH_2-CH_2-COOCH_3$ | |
| 1.105 | $CH(Br)CH(Br)COOCH_3$ | |
| 1.106 | $C(NH_2)=NOH$ | Smp. 209-211°C |
| 1.107 | $COCCl_3$ | $n_D^{26} = 1.6178$ |
| 1.108 | $CH(OH)CCl_3$ | Smp. 163-165°C |
| 1.109 | $CH=CH-COOCH_3$ | |
| 1.110 | $CH_2NH_2$ | Smp. 136-139°C |
| 1.111 | $CH=CH-CONH_2$ | |
| 1.112 | $C(OH)=NOCH_3$ | Smp. 188-190°C |
| 1.113 | $CH_2SCOCH_3$ | $n_D^{27}$ 1.6478 |
| 1.114 | $CH_2SCO-C_6H_5$ | |
| 1.115 | $CH_2NHCH_3$ | |
| 1.116 | $CH_2N(CH_3)_2$ | |
| 1.117 | $CH_2NH-C_6H_5$ | |
| 1.118 | $CH_2N(CH_3)C_6H_4-(4-CH_3)$ | |
| 1.119 | $CH_2NH(3,5-Di-Cl-C_6H_3)$ | |
| 1.120 | $CH(CH_3)NHC_6H_5$ | |
| 1.121 | $CH_2NH-NH-C_6H_5$ | |
| 1.122 | $CH_2NH-NH_2$ | |
| 1.123 | $CH_2NH-N(CH_3)_2$ | |
| 1.124 | $CH(Br)COOC_6H_5$ | |

| Verb. Nr. | A | Physik. Daten |
|---|---|---|
| 1.125 | $COCH_2Br$ | Smp. 155-157°C |
| 1.126 | $C(NH_2)=NH\cdot HCl$ | |
| 1.127 | $C(CN)=NOH$ | Smp. 249-253°C |
| 1.128 | $C(CN)=NOCH_3$ | |
| 1.129 | $C(OH)-CF_3$ | |
| 1.130 | $CO-CF_3$ | |
| 1.131 | $C(=NH)-N(CH_3)_2$ (Hydrogenoxalat) | |
| 1.132 | $C(=NH)NH(3,5\text{-di-Cl-}C_6H_3)\cdot HCl$ | |
| 1.133 | $CH(Cl)COOH$ | |
| 1.134 | $CH(Cl)COOCH_3$ | |
| 1.135 | $CH(Br)COOC_2H_5$ | |
| 1.136 | $C(Cl)=C(Cl)COOCH_3$ | |
| 1.137 | $C(Cl)=C(Cl)_2$ | |
| 1.138 | $C(NH_2)(CH_3)CN$ | |
| 1.139 | $C(CH_3)(NH_2)-COOCH_3$ | |
| 1.140 | $C(COOCH_3)(=CH-OH)$ | |
| 1.141 | $C(COOCH_3)(=CH-OCH_3)$ | |
| 1.142 | $C(CN)(=CH-OCH_3)$ | |
| 1.143 | $C(=NH)(NH-NH_2)$ | |
| 1.144 | $C(CH_3)-ON=C(CH_3)_2$ | |
| 1.145 | $CH=CH_2$ | |
| 1.146 | $CH\equiv CH-CH_3$ | |
| 1.147 | $CH=CCl_2$ | |
| 1.148 | $C(Cl)-CCl_3$ | |
| 1.149 | $CHBr-CH_2Br$ | |

Tabelle 2

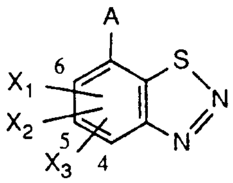

| Verb. Nr. | $X_1, X_2, X_3$ | A | Physik. Daten |
|---|---|---|---|
| 2.1 | 6-Cl | $CCl_3$ | |
| 2.2 | 6-F | $CF_3$ | |
| 2.3 | 5-F | $CF_3$ | |
| 2.4 | 6-Br | CHO | |
| 2.5 | 6-F | CHO | |
| 2.6 | 6-F | $CH_2O(CO)$-Phenyl | |
| 2.7 | 5-F | $CH_2OCOCH_3$ | |
| 2.8 | 4-F | $COCH_3$ | |
| 2.9 | 6-F | $COCH_3$ | |
| 2.10 | 4,5-Di-F | CHO | |
| 2.11 | 5-F | $CH_2OSO_2CH_3$ | |
| 2.12 | 6-F | $C(OCH_3)_3$ | |
| 2.13 | 5,6-Di-F | $CH_2COOH$ | |
| 2.14 | 4-F | $CH_2COS$-Phenyl | |
| 2.15 | 5-Br | CH(OH)COOH | |
| 2.16 | 6-Cl | $CH[OSi(CH_3)_3]CN$ | |
| 2.17 | 4-Cl | CH=NOH | |
| 2.18 | 5-$NO_2$ | $COCOOCH_3$ | |
| 2.19 | 4-$CH_3$ | $CH_2ON=C(CN)CONH_2$ | |
| 2.20 | 5-F | CH=N-2'-Cl-Benzyl | |
| 2.21 | 5-F | CH=CH-COOH | |
| 2.22 | 6-F | $C(NH_2)=NOCH_3$ | |
| 2.23 | 6-F | $CH_2NH$-Cyclopropyl | |
| 2.24 | 6-F | $CH_2NH-N(C_2H_5)_2$ | |
| 2.25 | 6-Br | CH(Br)COO-Benzyl | |
| 2.26 | 6-F | $CH_2OCO-(2'-OH)-C_6H_4$ | |

| Verb. Nr. | $X_1, X_2, X_3$ | A | Physik. Daten |
|---|---|---|---|
| 2.27 | 6-F | $CH(OH)CH_3$ | |
| 2.28 | 6-$CH_3$ | CHO | |
| 2.29 | 4,6-Di-$CH_3$ | CHO | |
| 2.30 | 5-$CH_3$ | $CH_2OCH_3$ | |
| 2.31 | 5-$CH_3$ | $CH_2OCOCH_3$ | |
| 2.32 | 5-F | CHO | Smp. 129-131°C |
| 2.33 | 6-$SCH_3$ | $COCH_3$ | |
| 2.34 | 5-$SCH_3$ | CHO | |
| 2.35 | 5-F | $CH(OH)CCl_3$ | Smp. 148-150°C |
| 2.36 | 5-F | $CH_2O(CO)$-Cyclopropyl | Smp. 74-76°C |
| 2.37 | 4,6-Di-F | CHO | |
| 2.38 | 6-F | $CH_2CN$ | |
| 2.39 | 6-F | $CH(NH_2)CN$ | |
| 2.40 | 6-F | $C(N(CH_3)_2)=NH$ | |
| 2.41 | 5-F | $C(COOCH_3)=CH-OH$ | |
| 2.42 | 5-F | $C(COOCH_3=CH-OMe$ | |
| 2.43 | 5-F | $C(=NOH)NH_2$ | |
| 2.44 | 4-F | CHO | |
| 2.45 | 5-F | $C(=NOH)CN$ | |
| 2.46 | 5-F | $C\equiv CH$ | |
| 2.47 | 5-F | $CH(Cl)CCl_3$ | |
| 2.48 | 6-F | $CH=CH_2$ | |
| 2.49 | 4-F | $CH=CCl_2$ | |

Tabelle 3

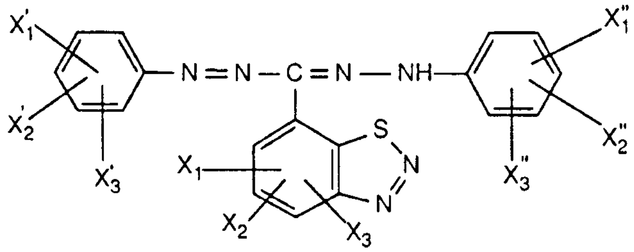

| Verb. Nr. | $X_1$, $X_2$, $X_3$ | $X_1'$, $X_2'$, $X_3'$ | $X_1''$, $X_2''$, $X_3''$ | Physik. Daten Smp. |
|---|---|---|---|---|
| 3.1 | - | - | - | 185-187°C |
| 3.2 | - | 4-Cl | 4-Cl | |
| 3.3 | - | 4-$NO_2$ | - | |
| 3.4 | - | 3,5-di-Cl | - | |
| 3.5 | - | - | 2,4,6-triCl | |
| 3.6 | - | 3-$CF_3$ | - | |
| 3.7 | - | 3,5-di-$CF_3$ | 2F | |
| 3.8 | - | - | 3$CF_3$ | |
| 3.9 | - | - | 2,4-di-$NO_2$ | |
| 3.10 | - | 2-Me | 2 Br | |
| 3.11 | - | - | 2,6-di-Br | |
| 3.12 | - | 2-Cl | 4-Me | |
| 3.13 | 4-F | - | - | |
| 3.14 | 5-F | - | - | |
| 3.15 | 6-F | - | - | |
| 3.16 | 6-F | - | 2,4,6-tri-Cl | |
| 3.17 | 4,6-di-F | - | - | |
| 3.18 | 5,6-di-F | - | - | |
| 3.19 | 4,5-diF | - | - | |
| 3.20 | 4,5,6-triF | - | - | |
| 3.21 | 5-Br | 4-$NO_2$ | 2-Cl | |
| 3.22 | 5-$NO_2$ | - | - | |
| 3.23 | 6-OMe | 3-$CF_3$ | 3,5-di-Br | |
| 3.24 | 6-SMe | - | 6-$NO_2$ | |

Tabelle 4

Literaturbekannte Verbindungen, deren Verwendung als Fungizide neu ist

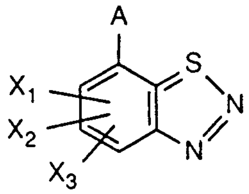

| Verb. Nr. | $X_1, X_2, X_3$ | A | Physik. Daten |
|---|---|---|---|
| 4.1 | - | CHO | Smp. 134-138°C |
| 4.2 | - | $COCH_3$ | Smp. 126-127°C |
| 4.3 | 6-Cl | CHO | Smp. 148-150°C |
| 4.4 | 6-SMe | CHO | Smp. 157-158°C |
| 4.5 | 4-Br-6Cl | CHO | Smp. 131-133°C |
| 4.6 | 6-OMe | CHO | Smp. 174-176°C |
| 4.7 | - | CH=NOH | Smp. 230°C |
| 4.8 | 6-OMe | CH=NOH | Smp. 217-218°C |
| 4.9 | - | $COCHBr_2$ | Smp. 119-120°C |

Formulierungsbeispiele für Wirkstoffe aus den Tabellen (% = Gewichtsprozent)

2.1 Spritzpulver

| | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen.

Man erhält Spritzpulver, die sich mit Wasser zu Suspensionen jeder gewünschten Konzentration verdünnen lassen.

2.2 Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

<u>2.3 Stäubemittel</u>

| | a) | b) |
|---|---|---|
| Wirkstoff aus den Tabellen | 5 % | 8 % |
| Talkum | 95 % | - |
| Kaolin | - | 92% |

Man erhält anwendungsfertige Stäubemittel, indem der Wirkstoff mit den Trägerstoffen vermischt und auf einer geeigneten Mühle vermahlen wird.

2.4 Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5 Umhüllungs-Granulat

| | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200ppm). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C bis 23°C weitergeführt.
Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen). Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C weitergeführt.
Die Beurteilung der Schutzwirkung erfolgt aufgrund des Pilzbefalls 7-8 Tage nach der Infektion.

c) Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei

normaler Luftfeuchigkeit und bei 22° bis 23°C weitergeführt.

Die Beurteilung der Schutzwirkung erfolgt aufgrund des Pilzbefalls 7-8 Tage nach der Infektion.

Verbindungen aus den Tabellen 1 bis 4 zeigen in den Tests (a) und (b) gute Wirkung. So reduzieren z.B. die Verbindungen 1.2, 1.6, 1.7, 1.12, 1.33, 1.38, 1.64, 1.79, 1.83, 1.85, 1.89, 1.91, 1.106, 1.108, 1.110, 1.113, 1.125, 1.127, 3.1, 4.1 und 4.2 den Pilzbefall auf 0 bis 20%. Unbehandelte jedoch infizierte Kontrollpflanzen weisen dagegen einen Colletotrichum-Befall von 100% auf.

Beispiel 3.2: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02% Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation derinfizierten Pflanzen während 5 Tagen bei 9O-100% relativer Luftfeuchtigkeit und 20°C.

b) Zu Tomatenpflanzen wird nach 3-wöchiger Anzucht eine aus Spritzpulverdes Wirkstoffes hergestellte Spritzbrühe gegossen (0,006% Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100% relativer Luftfeuchtigkeit und 20°C.

Verbindungen aus den Tabellen 1 bis 4 zeigen gegen den Phytophthora-Pilz eine gute Schutzwirkung. Soreduzieren z.B. im Test a) die Verbindungen 1.6, 1.31, 1.38, 1.83, 1.127 und 4.1 und im Test b) 1.1, 1.6, 1.12, 1.31, 1.38, 1.83, 1.85, 1.101 und 1.106 den Pilzbefall auf 0 bis 20%. Unbehandelte jedoch infizierte Kontrollpflanzen weisen dagegen einen 100 %igen Phytophthora-Befall auf.

Beispiel 3.3: Wirkung gegen Pyricularia oryzae auf Reispflanzen

a) Reispflanzen werden nach 2-wöchiger Anzucht mit einer aus Spritzpulver des Wirkstoffes hergestellten Spritzbriihe (0,02% Aktivsubstanz) besprüht. Nach 48 Stunden werden die behandelten Pflanzen mit einer Konidiensuspension des Pilzes infiziert. Nach 5 Tagen Inkubation bei 95-100% relativer Luftfeuchtigkeit und 24°C wird der Pilzbefall beurteilt.

b) Zu 2-wöchigen Reispflanzen wird eine aus Spritzpulver des Wirkstoffes hergestellte Spritzbrühe gegossen (0,006% Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100% relativer Luftfeuchtigkeit und ca. 24°C wird der Pilzbefall beurteilt.

Reispflanzen, die mit einer Spritzbrühe behandelt werden, die als Aktivsubstanz eine Verbindung aus den Tabellen 1 bis 4 enthält, zeigen im Vergleich zu unbehandelten Kontrollpflanzen (100 % Befall) nur geringen Pilzbefall. Soreduzierten z.B. im Test (a) die Verbindungen 1.6, 1.98, 1.101 und 4.1 und im Test (b) die Verbindungen 1.6, 1.64, 1.66, 1.101, 4.1 und 4.7 den Befall auf 0 bis 20 %.

Beispiel 3.4: Wirkung gegen Peronospora tabacina an Tabakpflanzen

A) Blattapplikation

Tabakpflanzen (8 Wochen alt) werden mit einer formulierten Lösung des Wirkungsstoffes besprüht (Konzentration: 0,02% Aktivsubstanz). Vier Tage nach der Behandlung werden die Pflanzen mit einer Sporangiensuspension von Peronospora tabacina ($10^4$ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

B) Bodenapplikation

Tabakpflanzen (8 Wochen alt) werden mit einer formulierten Lösung des Wirkungsstoffes durch Bodenapplikation behandelt (Konzentration: 0,006% Aktivsubstanz bezogen auf das Bodenvolumen). Nach 4 Tagen werden die

Pflanzen mit einer Sporangiensuspension von Peronospora tabacina ($10^4$ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

Die Beurteilung der Symptome in den Tests A und B erfolgt aufgrund der mit Pilz befallenen Blattoberfläche.

Die Kontrollpflanzen zeigen einen Befall von 90 bis 100%.Pflanzen, welche in Test A mit der Verbindung 1.6 oder 4.1 behandelt wurden, zeigten einen Befall von 0-30%.

<u>Beispiel 3.5</u>: <u>Wirkung gegen Bremia lactucae auf Salat</u>

Zu zweiwöchigen Salatpflanzen wird eine formulierte Lösung des Wirkstoffes (0,002% Aktivsubstanz bezogen auf das Erdvolumen) gegossen. Nach 5 Tagen werden die behandelten Pflanzen mit einer Sporensuspension des Pilzes (5 x $10^4$ s/ml) inokuliert. Die Pflanzen werden bei 18°C zuerst unter einer Haube (relative Luftfeuchtigkeit von 90-100%) während 2 Tagen, dann ohne Haube während 7 Tagen inkubiert. Um den Pilz zur Sporulation zu bringen, werden die Pflanzen wieder für 3 Tage unter eine Haube gestellt.

Die Beurteilung des Pilzbefalls erfolgt 12 Tage nach der Inokulation aufgrund der mit Pilz befallenen Blattoberfläche.

Verbindungen aus den Tabellen 1 bis 4 zeigen eine gute Wirkung gegen Bremia. So blieben Pflanzen, die z.B. mit den Verbindungen 1.1, 1.2, 1.6, 1.98 oder 4.1 behandelt wurden, weitgehend befallsfrei (0-30% Schädigung). Unbehandelte, aber infizierte Pflanzen (Kontrolle) wiesen dagegen einen Bremia-Befall von 100% auf.

<u>Beispiel 3.6</u>: <u>Wirkung gegen Erysiphe graminis auf Weizen</u>

<u>Protektive Wirkung:</u> 17 Tage alte Weizenpflanzen werden mit einer formulierten Lösung des Wirkstoffes (0,02 % Aktivsubstanz) besprüht. Unmittelbar nach der Behandlung werden die Pflanzen unter Zylindern inkubiert. Nach 24 Stunden werden die Pflanzen abgedeckt. Nach weiteren 3 Tagen werden die behandelten Pflanzen oberhalb vom Primärblatt abgeschnitten. Die Primärblätter werden horizontal orientiert und in einer Bestäubungsglocke mit Eryiphe graminis-Sporen inokuliert (Sporendichte: 0,2 mg pro $m^2$). Der Test wird in einer Klimakammer bei 12h Licht (18 KLux), 20°C und 12h Dunkelheit, 18°C durchgeführt.

Die Beurteilung des Befalls erfolgt 9 und 13 Tage nach Inokulation.

Als Wirkstoff eingesetzte Verbindungen aus den Tabellen 1 bis 4 zeigen in diesem Test eine gute Wirkung gegen Erysiphe graminis. So bleiben Pflanzen, die z.B. mit der Verbindung 1.1, 1.2, 1.6, 1.7, 1.12, 1.31, 1.33, 1.38, 1.66, 1.83, 1.98, 1.108, 4.1 oder 4.2 behandelt wurden, weitgehend frei von Erysiphe-Befall (0 bis 20% Schädigung). Unbehandelte aber infizierte Pflanzen (Kontrolle) weisen dagegen einen Erysiphe-Befall von 100 % auf.

<u>Beispiel 3.7</u>: <u>Wirkung gegen Cercospora arachidicola auf Erdnusspflanzen</u>

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen 1 bis 4 behandelt wurden, einen stark reduzierten Cercospora-Befall. So verhinderten die Verbindungen Nr. 1.83, 1.85, 1.108,1.125, 1.127, 3.1 und 4.7 in obigen Versuchen das Auftreten von Flecken weitgehend (0-20 %).

<u>Beispiel 3.8</u>: <u>Wirkung gegen Puccinia graminis auf Weizen</u>

a) <u>Residual-protektive Wirkung</u>

Weizenpflanzen werden 6 Tage nach der Aussaat mit einer aus Spritzpulver des Wirkstoffes hergestellten Spritzbriihe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006% Aktivsubstanz bezogen aufdas Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.
Unbehandelte aber infizierte Kontrollpflanzen zeigen einen Puccinia-Befall von 100 %. Verbindungen aus den Tabellen 1 bis 4 zeigen gegen Puccinia-Pilze eine gute Wirkung.
So reduzieren z.B. im Test a) die Verbindungen Nr. 1.7, 1.8, 1.31, 1.64, 1.79, 1.83, 1.98, 1.113, 1.125, 1.127 und im Test b) Nr. 1.113 den Pilzbefall auf unter 20 %.

Beispiel 3.9: Wirkung gegen Pseudomonas lachrymans an Cucumis sativus L.

A) Blattapplikation

Gurkenpflanzen werden nach 2-wöchiger Anzucht mit einer aus Spritzpulver des Wirkstoffes hergestellten Spritzbrühe besprüht (Konzentration: 0,02 % Aktivsubstanz).
Nach 1 Woche werden die Pflanzen mit einer Bakteriensuspension ($10^8$ Bakterien/ml) infiziert und für 7 Tage bei hoher Luftfeuchtigkeit und einer Temperatur von 23°C inkubiert.
Die Beurteilung der Schutzwirkung erfolgt aufgrund des Bakterienbefalls 7-8 Tage nach der Infektion. Verbindungen aus den Tabellen 1 bis 4 bewirken einen guten Schutz gegen Pseudomonas lachrymans. So blieben Pflanzen, die z.B. mit der Verbindung 1.31, 1.38, 1.108 oder 4.2 behandelt wurden, weitgehend frei von Pseudomonas (Befall 20 bis 0 %).

B) Bodenapplikation

Gurkenpflanzen werden nach 2-wöchiger Anzucht mit einer aus Spritzpulver des Wirkstoffes hergestellten Spritzbrühe durch Bodenapplikation behandelt (Konzentration: 0,002 % Aktivsubstanz bezogen auf das Bodenvolumen).
Nach 1 Woche werden die Pflanzen mit einer Bakteriensuspension ($10^8$ Bakterien/ml) infiziert und für 7 Tage bei hoher Luftfeuchtigkeit und einer Temperatur von 23°C inkubiert.
Die Beurteilung der Schutzwirkung erfolgt aufgrund des Befalls 7-8 Tage nach der Infektion.
Verbindungen aus den Tabellen 1 bis 4 bewirken eine gute Immunisierung gegen Pseudomonas lachrymans. So blieben Pflanzen, die z.B. mit der Verbindung 1.31, 1.33, 1.38 oder 1.108 behandelt wurden, fast völlig frei von Pseudomonas (Befall 20 bis 0 %).
Unbehandelte aber infizierte Kontrollpflanzen weisen in den Tests A und B einen Krankheitsbefall von 100 % auf.

Beispiel 3.10: Wirkung gegen Plasmopara viticola an Reben

Im 4-5 Blattstadium werden Rebensämlinge mit einer aus Spritzpulver des Wirkstoffes hergestellten Spritzbrühe (0,02 % Aktivsubstanz) besprüht. Nach 1 Woche werden die behandelten Pflanzen mit einer Sporangiensuspension $5 \cdot 10^4$ Sporangien/ml) des Pilzes infiziert. Nach einer Inkubation während 6 Tagen bei 95-100 % relativer Luftfeuchtigkeit und 20°C wird die Schutzwirkung beurteilt.
Unbehandelte aber infizierte Kontrollpflanzen weisen in diesem Test einen Befall von 100 % auf.
Verbindungen aus den Tabellen 1 bis 4 bewirken eine gute Immunisierung gegen Plasmopara viticola. So blieben Reben, die z.B. mit der Verbindung 1.83 oder 1.125 behandelt wurden, weitgehend frei von Plasmopara viticola (Befall 20 bis 0 %).

Beispiel 3.11: Wirkung gegen Cercospora nicotianae an Tabakpflanzen Blattapplikation

Tabakpflanzen (8 Wochen alt) werden mit einer fomulierten Lösung des Wirkstoffes besprüht (Konzentration: 200 ppm). Vier Tage nach der Behandlung werden die Pflanzen mit einer Sporensuspension von Cercospora nicotianae ($10^5$ Sporen/ml) inokuliert und für 5 Tage bei hoher Luftfeuchtigkeit und einer Temperatur von 22°-25°C inkubiert. Die Inkubation wird dann bei normaler Luftfeuchtigkeit und bei 20°-22°C weitergeführt.
Die Beurteilung der Symptome in den Tests erfolgt aufgrund des Pilzbefalls 12-14 Tage nach der Infektion.
Die Kontrollpflanzen zeigten einen Befall von 100 %. Pflanzen, welche mit den Verbindungen 1.83, 1.85,

1.108, 1.125, 1.127, 3.1 oder 4.7 behandelt wurden, zeigten einen Befall von 0-20 %.

Beispiel 3.12: Wirkung gegen Pythium ultimum auf Zea mays (Mais, cv. Sweet Corn)

Testprinzip: Bodenpilz: protektiv lokale Bodenapplikation.

Testmethode: Myzel von Pythium ultimum wird mit Erde gemischt (500 ml Myzelsuspension auf 10 Liter Erde) und das Pilz-Erdgemisch in 250 ml Plastikschalen abgefüllt. Nach einer 4-tägigen Inkubation bei 10°C werden pro Schale 10 Körner der zu prüfenden Pflanze (Mais) gesteckt. Am nächsten Tag werden die so vorbereiteten Schalen mit je 50 ml, aus 25% Spritzpulver und Wasser hergestellten Spritzlösungen mit 20; 6; 2; 0,6; 0,2; 0,06 und 0,02 ppm AS übergossen. Nach einer 7-tägigen Inkubationsphase bei 10°C und einer anschliessenden 4-tägigen Inkubationsphase bei 22°C wird die Wirkung der Testsubstanzen durch zahlenmässige Bestimmung des Auflaufs der Testpflanzen bewertet.
Verbindungen aus den Tabellen 1-4 erzielen gegen Pythium ultimum gute Wirkung. So zeigte die Verbindung 1.125 eine Wirkung von mehr als 80 %.

**Patentansprüche**

1. Verwendung einer Verbindung der Formel I zur Bekämpfung oder Verhütung eines Befalls durch schädliche Mikroorganismen,

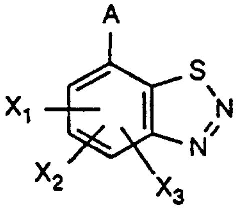

(I)

worin bedeuten:

$X_1$, $X_2$ und $X_3$ Wasserstoff,
A durch maximal 3 X-$C_1$-$C_4$-Alkylgruppen substituiertes $C_1$-$C_2$-Alkyl, durch 2 oder 3 Halogenatome substituiertes Methyl, durch Hydroxy und/oder bis zu 4 Halogenatome substituiertes Ethyl, unsubstituiertes oder bis zu 3 Halogenatome tragendes Vinyl; ferner Ethinyl, Propargyl, Formyl, Acetyl, durch maximal 3 Halogenatome substituiertes Acetyl oder eine der Gruppen $C(R)=N-N(R_2)R_3$, $C(N=N-U_1)=N-NH-U_1$, $CH(R)-[N(R_1)]_n-N(R_2)R_3$, $C(R)(CN)OR_4$, $C(R)=N(O)_nR_3$, $CH(R)-O-N=C(R_1)R_2$, $CH(R)-O-N=C(CN)-CONH-R_5$, $C(R)_6=N(O)_nR$, $CH(R)-Y-E-R_3$, $CO-[C(OR)_2]_nQ$, C(Q)=CH-OR oder T-Q;
worin ferner bedeuten:
n Null oder 1;
X und Y unabhängig voneinander Sauerstoff oder Schwefel;
R und $R_1$ unabhängig voneinander Wasserstoff oder $C_1$-$C_2$-Alkyl;
$R_2$ Wasserstoff, $C_1$-$C_8$-Alkyl, $C_3$-$C_6$-Alkenyl, $C_3$-$C_6$-Alkinyl, $C_3$-$C_7$-Cycloalkyl, Benzyl oder Cyano;
$R_3$ Wasserstoff, $C_1$-$C_6$-Alkyl, $C_3$-$C_6$-Alkenyl, $C_3$-$C_6$-Alkinyl, $C_3$-$C_7$-Cycloalkyl, Benzyl oder einen Arylrest U;
$R_4$ Wasserstoff, $C_1$-$C_6$-Alkyl, $Si(C_1$-$C_6$-$Alkyl)_3$ oder $OCOC_1$-$C_3$-Alkyl;
$R_5$ Wasserstoff oder $CONHR_1$;
$R_6$ $N(R_1)R_2$, Hydrazino oder Q:
E CO oder $SO_2$;
U und $U_1$ unabhängig voneinander einen unsubstituierten oder durch Methyl, Methoxy, Halogen,Trifluormethyl, Nitro oder Cyano ein- oder mehrfach gleich oder verschieden substituierten Phenylrest;
T $C_1$-$C_2$-Alkylen, unabhängig voneinander durch Amino, Hydroxy oder Halogen substituiertes Methylen, unsubstituiertes oder durch Halogen oder Cyano substituiertes Ethenylen;
Q COXR oder Cyano.

**2.** Verwendung gemäss Anspruch 1, dadurch gekennzeichnet, dass in Verbindung der Formel I, bedeuten:

$X_1$, $X_2$ und $X_3$ Wasserstoff;
A durch maximal 3 X-$C_1$-$C_4$-Alkylgruppen substituiertes $C_1$-$C_2$-Alkyl, durch 2 oder 3 Halogenatome substituiertes Methyl, durch Hydroxy und/oder bis zu 4 Halogenatome substituiertes Ethyl; oder Formyl, Acetyl oder eine der Gruppen $C(R)=N-N(R_2)R_3$, $CH(R)-[N(R_1)]_n-N(R_2)R_3$, $C(R)(CN)OR_4$, $C(R)=N(O)_nR_3$, $CH(R)-O-N=C(R_1)R_2$, $CH(R)-O-N=C(CN)-CONH-R_5$, $C(R_6)=N-(O)_nR$, $CH(R)-Y-E-R_3$, $CO-[C(OR)_2]_nCOXR$, $C(Q)=CH-OR$ oder T-Q;
worin ferner bedeuten:
n Null oder 1;
X und Y Sauerstoff;
R und $R_1$ unabhängig voneinander Wasserstoff oder $C_1$-$C_2$-Alkyl;
$R_2$ Wasserstoff, $C_1$-$C_4$-Alkyl, $C_3$-$C_4$-Alkenyl, $C_3$-$C_4$-Alkinyl, $C_3$-$C_6$-Cycloalkyl oder Benzyl;
$R_3$ Wasserstoff, $C_1$-$C_4$-Alkyl, $C_3$-$C_4$-Alkenyl, $C_3$-$C_4$-Alkinyl, $C_3$-$C_6$-Cycloalkyl oder einen durch Methyl, Halogen oder Trifluormethyl gleich oder verschieden substituierten Phenylrest;
$R_4$Wasserstoff, $C_1$-$C_3$-Alkyl oder $Si(C_1$-$C_2$-$Alkyl)_3$;
$R_5$ Wasserstoff oder $CONHC_1$-$C_3$-Alkyl;
$R_6$ Amino oder Cyano;
E CO;
T Methylen, durch Amino substituiertes Methylen oder Ethenylen;
Q COOR oder Cyano.

**3.** Verwendung gemäss Anspruch 1, dadurch gekennzeichnet, dass in Verbindung der Formel I, bedeuten:

$X_1$, $X_2$ und $X_3$ Wasserstoff;
A durch maximal 2 X-$C_1$-$C_4$-Alkylgruppen substituiertes Methyl, durch 2 oder 3 Fluor- oder Chloratome substituiertes Methyl, durch Hydroxy oder Chlor substituiertes Ethyl, oder Formyl, Acetyl oder eine der Gruppen $C(R)=N-N(R_2)R_3$, $CH(R)-[N(R_1)]_n-N(R_2)R_3$, $C(R)(CN)OR_4$, $C(R)=N(O)_nR_3$, $CH(R)-O-N=C(R_1)R_2$, $CH(R)-O-N=C(CN)-CONHR_5$, $C(R_6)=N-OR$, $CH(R)-Y-E-R_3$, $CO[C(OR)_2]_nCOXR$, $C(COOR)=CH-OR$ oder T-Q;
worin ferner bedeuten:
n 1;
X Sauerstoff;
R und $R_1$ unabhängig voneinander Wasserstoff oder Methyl;
$R_2$ Wasserstoff, $C_1$-$C_2$-Alkyl, Allyl, Propargyl, Cyclopropyl oder Benzyl;
$R_3$ Wasserstoff, $C_1$-$C_2$-Alkyl, Allyl, Propargyl, Cyclopropyl oder einen durch Methyl, Fluor, Chlor oder Trifluormethyl gleich oder verschieden subtituierten Phenylrest;
$R_4$ Wasserstoff, $C_1$-$C_2$-Alkyl oder $Si(CH_3)_3$;
$R_5$ Wasserstoff oder CONH-$C_1$-$C_2$-Alkyl;
$R_6$ Amino;
Y Sauerstoff;
E CO;
T Methylen oder Cyano.

**4.** Verwendung gemäss Anspruch 1, dadurch gekennzeichnet, dass in Verbindung der Formel I, bedeuten:

$X_1$, $X_2$ und $X_3$ Wasserstoff;
A durch maximal 2 X-$C_1$-$C_2$-Alkylgruppen substituiertes Methyl, durch 2 oder 3 Fluoratome substituiertes Methyl, durch Hydroxy oder Chlor substituiertes Ethyl, oder Formyl, oder eine der Gruppen $C(R)=N-N(R_2)R_3$, $CH(R)-[N(R_1)_n-N(R_2)R_3$, $C(R)(CN)OR_4$, $C(R)=N-R_3$, $CH(R)-O-N=C(R_1)R_2$, $C(R_6)=N-OR$, $CH(R)-Z-E-R_3$, $CO[C(OR)_2]_nCOXR$ oderT-Q;
worin ferner bedeuten:
n 1;
X Sauerstoff;
R und $R_1$ unabhängig voneinander Wasserstoff oder Methyl;
$R_2$ Wasserstoff, Methyl, Allyl, Cyclopropyl oder Benzyl;
$R_3$ Wasserstoff, Methyl, Allyl, Cyclopropyl oder einen durch Methyl, Fluor, Chlor oder Trifluormethyl gleich oder verschieden subtituierten Phenylrest;
$R_4$ Wasserstoff, Methyl oder $Si(CH_3)_3$;

$R_5$ Wasserstoff oder CONH-$CH_2$-$CH_3$;
$R_6$ Amino;
Y Sauerstoff;
E CO;
T Methylen;
Q $COOCH_3$ oder Cyano; enthält.

**5.** Verwendung gemäss Anspruch 1, dadurch gekennzeichnet, dass die Verbindung aus der Gruppe:

7-Formyl-1,2,3-benzothiadiazol;
7-Acetoxymethyl-1,2,3-benzothiadiazol;
7-[Methoxyimino-(2-cyanoacetamyl)]-1,2,3-benzothiadiazol;
7-(N-Methoxyimino-methyl)-1,2,3-benzothiadiazol;
7-(N-Methoxyimino-hydroxymethyl)-1,2,3-benzothiadiazol;
7-Methoxymethyl-1,2,3-benzothiadiazol;
3-(7-Benzo-1,2,3-thiadiazol)-acrylsäure;
7-Cyanomethyl-1,2,3-benzothiadiazol;
7-Trichlormethyl-1,2,3-benzothiadiazol;
7-Dichlormethyl-1,2,3-benzothiadiazol;
Benz-1,2,3-thiadiazol-7-(N-hydroxycarboximid-amid);
Benz-1,2,3-thiadiazol-7-(N-methoxyhydroxamsäure);
2-(Benz-1,2,3-thiadiazolyl)-2-hydroxyiminoacetonitril;
5-Fluor-benz-1,2,3,-thiadiazol-7-carbaldehyd;
6-Fluor-benz-1,2,3-thiadiazol-7-carbaldehyd;
4-Fluor-benz-1,2,3-thiadiazol-7-carbaldehyd;
N,N-Diphenyl-C-[benz-1,2,3-thiadiazol-7'yl]formazan;
7-Acetyl-benz-1,2,3-thiadiazol;
7-(Bromacetyl)-benz-1,2,3-thiadiazol ausgewählt ist.

**6.** Verbindungen der Formel I gemäss Anspruch 1 mit Ausnahme folgender Verbindungen:

7-Formyl-1,2,3-benzothiadiazol;
7-Acetyl-1,2,3-benzothiadiazol;
6-Chlor-7-formyl-1,2,3-benzothiadiazol;
6-Methylthio-7-formyl-1,2,3-benzothiadiazol;
4-Brom-6-chlor-7-formyl-1,2,3-benzothiadiazol;
6-Methoxy-7-formyl-1,2,3-benzothiadiazol;
7-Hydroxyimino-1,2,3-benzothiadiazol;
6-Methoxy-7-oximino-1,2,3-benzothiadiazol.
7-Dibromacetyl-1,2,3-benzothiadiazol.

**7.** Verbindungen der Formel I gemäss Anspruch 2 mit Ausnahme folgender Verbindungen:

7-Formyl-1,2,3-benzothiadiazol;
7-Acetyl-1,2,3-benzothiadiazol;
6-Chlor-7-formyl-1,2,3-benzothiadiazol;
6-Methylthio-7-formyl-1,2,3-benzothiadiazol;
4-Brom-6-chlor-7-formyl-1,2,3-benzothiadiazol;
6-Methoxy-7-formyl-1,2,3-benzothiadiazol;
7-Hydroxyimino-1,2,3-benzothiadiazol;
6-Methoxy-7-oximino-1,2,3-benzothiadiazol.
7-Dibromacetyl-1,2,3-benzothiadiazol.

**8.** Verbindungen der Formel I gemäss Anspruch 3 mit Ausnahme folgender Verbindungen:

7-Formyl-1,2,3-benzothiadiazol;
7-Acetyl-1,2,3-benzothiadiazol;
6-Chlor-7-formyl-1,2,3-benzothiadiazol;

6-Methylthio-7-formyl-1,2,3-benzothiadiazol;
4-Brom-6-chlor-7-formyl-1,2,3-benzothiadiazol;
6-Methoxy-7-formyl-1,2,3-benzothiadiazol;
7-Hydroxyimino-1,2,3-benzothiadiazol;
6-Methoxy-7-oximino-1,2,3-benzothiadiazol.
7-Dibromacetyl-1,2,3-benzothiadiazol.

**9.** Verbindungen der Formel I gemäss Anspruch 4 mit Ausnahme folgender Verbindungen:

7-Formyl-1,2,3-benzothiadiazol;
7-Acetyl-1,2,3-benzothiadiazol;
6-Chlor-7-formyl-1,2,3,-benzothiadiazol;
6-Methylthio-7-formyl-1,2,3-benzothiadiazol;
4-Brom-6-chlor-7-formyl-1,2,3-benzothiadiazol;
6-Methoxy-7-formyl-1,2,3-benzothiadiazol;
7-Hydroxyimino-1,2,3-benzothiadiazol;
6-Methoxy-7-oximino-1,2,3-benzothiadiazol.
7-Dibromacetyl-1,2,3-benzothiadiazol.

**10.** Eine Verbindung der Formel I ausgewählt aus der Gruppe:

7-Acetoxymethyl-1,2,3-benzothiadiazol;
7-[Methoxyimino-(2-cyanoacetamyl)]-1,2,3-benzothiadiazol;
7-(N-Methoxyimino-methyl)-1,2,3-benzothiadiazol;
7-(N-Methoxyimino-hydroxymethyl)-1,2,3-benzothiadiazol;
7-Methoxymethyl-1,2,3-benzothiadiazol;
3-(7-Benzo-1,2,3-thiadiazol)-acrylsäure;
7-Cyanomethyl-1,2,3-benzothiadiazol;
7-Trichlormethyl-1,2,3-benzothiadiazol;
7-Dichlormethyl-1,2,3-benzothiadiazol;
Benz-1,2,3-thiadiazol-7-(N-hydroxycarboximid-amid);
Benz-1,2,3-thiadiazol-7-(N-methoxyhydroxamsäure);
2-(Benz-1,2,3-thiadiazolyl)-2-hydroxyiminoacetonitril;
5-Fluor-benz-1,2,3,-thiadiazol-7-carbaldehyd;
6-Fluor-benz-1,2,3-thiadiazol-7-carbaldehyd;
4-Fluor-benz-1,2,3-thiadiazol-7-carbaldehyd;
N,N-Diphenyl-C-[benz-1,2,3-thiadiazol-7'yl]formazan;
7-(Bromacetyl)-benz-1,2,3-thiadiazol.

**11.** Verwendung gemäss Anspruch 1, dadurch gekennzeichnet, dass Nutzpflanzen gegen Krankheiten immunisiert werden.

**Claims**

**1.** The use of a compound of the formula I for controlling or preventing infestation by harmful microorganisms,

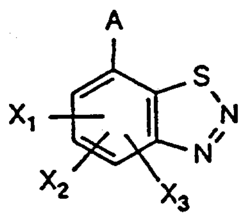

(I)

in which;

$X_1$, $X_2$ and $X_3$ are hydrogen:
A is $C_1$-$C_2$alkyl which is substituted by a maximum of 3 X-$C_1$-$C_4$alkyl groups, methyl which is substituted by 2 or 3 halogen atoms, ethyl which is substituted by hydroxyl and/or not more than 4 halogen atoms, vinyl which is unsubstituted or substituted by not more than 3 halogen atoms; furthermore ethynyl, propargyl, formyl, acetyl, acetyl which is substituted by not more than 3 halogen atoms, or one of the groups C(R)=N-N($R_2$)$R_3$, C(N=N-$U_1$)=N-NH-$U_1$, CH(R)-[N($R_1$)]$_n$-N($R_2$)$R_3$, C(R)(CN)O$R_4$, C(R)=N(O)$_n$$R_3$, CH(R)-O-N=C($R_1$)$R_2$, CH(R)-O-N=C(CN)-CONH-$R_5$, C($R_6$)=N-(O)$_n$R, CH(R)-Y-E-$R_3$, CO-[C(OR)$_2$]$_n$Q, C(Q)=CH-OR or T-Q;
in which furthermore:
n is zero or 1;
X and Y independently of one another are oxygen or sulfur;
R and $R_1$ independently of one another are hydrogen or $C_1$-$C_2$alkyl;
$R_2$ is hydrogen, $C_1$-$C_8$alkyl, $C_3$-$C_6$alkenyl, $C_3$-$C_6$alkynyl, $C_3$-$C_7$cycloalkyl, benzyl or cyano;
$R_3$ is hydrogen, $C_1$-$C_6$alkyl, $C_3$-$C_6$alkenyl, $C_3$-$C_6$alkynyl, $C_3$-$C_7$cycloalkyl, benzyl or is an aryl radical U;
$R_4$ is hydrogen, $C_1$-$C_6$alkyl, Si($C_1$-$C_6$alkyl)$_3$ or OCO$C_1$-$C_3$alkyl;
$R_5$ is hydrogen or CONH$R_1$;
$R_6$ is N($R_1$)$R_2$, hydrazino or Q;
E is CO or $SO_2$;
U and $U_1$ independently of one another are a phenyl radical which is unsubstituted or monosubstituted or polysubstituted by identical or different substituents from the series comprising methyl, methoxy, halogen, trifluoromethyl, nitro or cyano;
T is $C_1$-$C_2$alkylene, methylene which is substituted by amino, hydroxyl or halogen, the substituents being independent of one another, or is ethenylene which is unsubstituted or substituted by halogen or cyano;
Q is COXR or cyano.

**2.** The use according to claim 1, wherein, in the compound of the formula I:

$X_1$, $X_2$ and $X_3$ are hydrogen;
A is $C_1$-$C_2$alkyl which is substituted by a maximum of 3 X-$C_1$-$C_4$alkyl groups, methyl which is substituted by 2 or 3 halogen atoms, ethyl which is substituted by hydroxyl and/or not more than 4 halogen atoms, or formyl, acetyl, or one of the groups C(R)=N-N($R_2$)$R_3$, CH(R)-[N($R_1$)]$_n$-N($R_2$)$R_3$, C(R)(CN)O$R_4$, C(R)=N(O)$_n$$R_3$, CH(R)-O-N=C($R_1$)$R_2$, CH(R)-O-N=C(CN)-CONH-$R_5$, C($R_6$)=N-(O)$_n$R, CH(R)-Y-E-$R_3$, CO-[C(OR)$_2$]$_n$COXR, C(Q)=CH-OR or T-Q;
and furthermore:
n is zero or 1;
X and Y are oxygen;
R and $R_1$ independently of one another are hydrogen or $C_1$-$C_2$alkyl;
$R_2$ is hydrogen, $C_1$-$C_4$alkyl, $C_3$-$C_4$alkenyl, $C_3$-$C_4$alkynyl, $C_3$-$C_6$cycloalkyl or benzyl;
$R_3$ is hydrogen, $C_1$-$C_4$alkyl, $C_3$-$C_4$alkenyl, $C_3$-$C_4$alkynyl, $C_3$-$C_6$cycloalkyl or a phenyl radical which is substituted by identical or different substituents from the series comprising methyl, halogen or trifluoromethyl;
$R_4$ is hydrogen, $C_1$-$C_3$alkyl or Si($C_1$-$C_2$alkyl)$_3$;
$R_5$ is hydrogen or CONH$C_1$-$C_3$alkyl;
$R_6$ is amino or cyano;
E is CO;
T is methylene, methylene which is substituted by amino or ethenylene;
Q is COOR or cyano.

**3.** The use according to claim 1, wherein, in the compound of the formula I:

$X_1$, $X_2$ and $X_3$ are hydrogen;
A is methyl which is substituted by a maximum of 2 X-$C_1$-$C_4$alkyl groups, methyl which is substituted by 2 or 3 fluorine or chlorine atoms, ethyl which is substituted by hydroxyl or chlorine, or formyl, acetyl, or one of the groups C(R)=N-N($R_2$)$R_3$, CH(R)-[N($R_1$)]$_n$-N($R_2$)$R_3$, C(R)(CN)O$R_4$, C(R)=N(O)$_n$$R_3$, CH(R)-O-N=C($R_1$)$R_2$, CH(R)-O-N=C(CN)-CONH$R_5$, C($R_6$)=N-OR, CH(R)-Y-E-$R_3$, CO-[C(OR)$_2$]$_n$COXR, C(COOR)=CH-OR or T-Q;
and furthermore:
n is 1;
X is oxygen;
R and $R_1$ independently of one another are hydrogen or methyl;
$R_2$ is hydrogen, $C_1$-$C_2$alkyl, allyl, propargyl, cyclopropyl or benzyl;

$R_3$ is hydrogen, $C_1$-$C_2$alkyl, allyl, propargyl, cyclopropyl, or a phenyl radical which is substituted by identical or different substituents from the series comprising methyl, fluorine, chlorine or trifluoromethyl;
$R_4$ is hydrogen, $C_1$-$C_2$alkyl or $Si(CH_3)_3$;
$R_5$ is hydrogen or CONH-$C_1$-$C_2$alkyl;
$R_6$ is amino;
Y is oxygen;
E is CO;
T is methylene or cyano.

**4.** The use according to claim 1, wherein, in the compound of the formula I:

$X_1$, $X_2$ and $X_3$ are hydrogen;
A is methyl which is substituted by a maximum of 2 X-$C_1$-$C_2$alkyl groups, methyl which is substituted by 2 or 3 fluorine atoms, ethyl which is substituted by hydroxyl or chlorine, or formyl, or one of the groups C(R)=N-N($R_2$)$R_3$, CH(R)-[N($R_1$)]$_n$-N($R_2$)$R_3$, C(R)(CN)O$R_4$, C(R)=N-$R_3$, CH(R)-O-N=C($R_1$)$R_2$, C($R_6$)=N-OR, CH(R)-Z-E-$R_3$, CO-[C(OR)$_2$]$_n$COXR or T-Q;
and furthermore:
n is 1;
X is oxygen;
R and $R_1$ independently of one another are hydrogen or methyl;
$R_2$ is hydrogen, methyl, allyl, cyclopropyl or benzyl;
$R_3$ is hydrogen, methyl, allyl, cyclopropyl, or a phenyl radical which is substituted by identical or different substituents from the series comprising methyl, fluorine, chlorine or trifluoromethyl;
$R_4$ is hydrogen, methyl or $Si(CH_3)_3$;
$R_5$ is hydrogen or CONH-$CH_2$-$CH_3$;
$R_6$ is amino;
Y is oxygen;
E is CO;
T is methylene;
Q is $COOCH_3$ or cyano.

**5.** The use according to claim 1, wherein the compound is selected from the group:

7-formyl-1,2,3-benzothiadiazole;
7-acetoxymethyl-1,2,3-benzothiadiazole;
7-[methoxyimino-(2-cyanoacetamidyl)]-1,2,3-benzothiadiazole;
7-(N-methoxyiminomethyl)-1,2,3-benzothiadiazole;
7-(N-methoxyiminohydroxymethyl)-1,2,3-benzothiadiazole;
7-methoxymethyl-1,2,3-benzothiadiazole;
3-(7-benzo-1,2,3-thiadiazolyl)acrylic acid;
7-cyanomethyl-1,2,3-benzothiadiazole;
7-trichloromethyl-1,2,3-benzothiadiazole;
7-dichloromethyl-1,2,3-benzothiadiazole;
benzo-1,2,3-thiadiazole-7-(N-hydroxycarboximidamide);
benzo-1,2,3-thiadiazole-7-(N-methoxyhydroxamic acid);
2-(benzo-1,2,3-thiadiazolyl)-2-hydroxyiminoacetonitrile;
5-fluoro-benzo-1,2,3-thiadiazole-7-carbaldehyde;
6-fluoro-benzo-1,2,3-thiadiazole-7-carbaldehyde;
4-fluoro-benzo-1,2,3-thiadiazole-7-carbaldehyde;
N,N-diphenyl-C-[benzo-1,2,3-thiadiazol-7'yl]formazan;
7-acetylbenzo-1,2,3-thiadiazole;
7-(bromoacetyl)benzo-1,2,3-thiadiazole.

**6.** A compound of the formula I according to claim 1, with the exception of the following compounds:

7-formyl-1,2,3-benzothiadiazole;
7-acetyl-1,2,3-benzothiadiazole;
6-chloro-7-formyl-1,2,3-benzothiadiazole;

6-methylthio-7-formyl-1,2,3-benzothiadiazole;
4-bromo-6-fluoro-7-formyl-1,2,3-benzothiadiazole;
6-methoxy-7-formyl-1,2,3-benzothiadiazole;
7-hydroxyimino-1,2,3-benzothiadiazole;
6-methoxy-7-oximino-1,2,3-benzothiadiazole,
7-dibromoacetyl-1,2,3-benzothiadiazole.

**7.** A compound of the formula I according to claim 2, with the exception of the following compounds:

7-formyl-1,2,3-benzothiadiazole;
7-acetyl-1,2,3-benzothiadiazole;
6-chloro-7-formyl-1,2,3-benzothiadiazole;
6-methylthio-7-formyl-1,2,3-benzothiadiazole;
4-bromo-6-chloro-7-formyl-1,2,3-benzothiadiazole;
6-methoxy-7-formyl-1,2,3-benzothiadiazole;
7-hydroxyimino-1,2,3-benzothiadiazole;
6-methoxy-7-oximino-1,2,3-benzothiadiazole,
7-dibromoacetyl-1,2,3-benzothiadiazole.

**8.** A compound of the formula I according to claim 3, with the exception of the following compounds:

7-formyl-1,2,3-benzothiadiazole;
7-acetyl-1,2,3-benzothiadiazole;
6-chloro-7-formyl-1,2,3-benzothiadiazole;
6-methylthio-7-formyl-1,2,3-benzothiadiazole;
4-bromo-6-chloro-7-formyl-1,2,3-benzothiadiazole;
6-methoxy-7-formyl-1,2,3-benzothiadiazole;
7-hydroxyimino-1,2,3-benzothiadiazole;
6-methoxy-7-oximino-1,2,3-benzothiadiazole,
7-dibromoacetyl-1,2,3-benzothiadiazole.

**9.** A compound of the formula I according to claim 4, with the exception of the following compounds:

7-formyl-1,2,3-benzothiadiazole;
7-acetyl-1,2,3-benzothiadiazole;
6-chloro-7-formyl-1,2,3-benzothiadiazole;
6-methylthio-7-formyl-1,2,3-benzothiadiazole;
4-bromo-6-chloro-7-formyl-1,2,3-benzothiadiazole;
6-methoxy-7-formyl-1,2,3-benzothiadiazole;
7-hydroxyimino-1,2,3-benzothiadiazole;
6-methoxy-7-oximino-1,2,3-benzothiadiazole,
7-dibromoacetyl-1,2,3-benzothiadiazole.

**10.** A compound of the formula I, selected from the group:

7-acetoxymethyl-1,2,3-benzothiadiazole;
7-[methoxyimino-(2-cyanoacetamidyl)]-1,2,3-benzothiadiazole;
7-(N-methoxyiminomethyl)-1,2,3-benzothiadiazole;
7-(N-methoxyiminohydroxymethyl)-1,2,3-benzothiadiazole;
7-methoxymethyl-1,2,3-benzothiadiazole;
3-(7-benzo-1,2,3-thiadiazolyl)acrylic acid;
7-cyanomethyl-1,2,3-benzothiadiazole;
7-trichloromethyl-1,2,3-benzothiadiazole;
7-dichloromethyl-1,2,3-benzothiadiazole;
benzo-1,2,3-thiadiazole-7-(N-hydroxycarboximideamide);
benzo-1,2,3-thiadiazole-7-(N-methoxyhydroxamic acid);
2-(benzo-1,2,3-thiadiazolyl)-2-hydroxyiminoacetonitrile;
5-fluoro-benzo-1,2,3-thiadiazole-7-carbaldehyde;

6-fluoro-benzo-1,2,3-thiadiazole-7-carbaldehyde;
4-fluoro-benzo-1,2,3-thiadiazole-7-carbaldehyde;
N,N-diphenyl-C-[benzo-1,2,3-thiadiazol-7'yl]formazan;
7-(bromoacetyl)benzo-1,2,3-thiadiazole.

**11.** The use according to claim 1, wherein useful plants are immunised against diseases.

**Revendications**

**1.** Utilisation d'un composé de formule I pour combattre ou prévenir une attaque par des micro-organismes nuisibles,

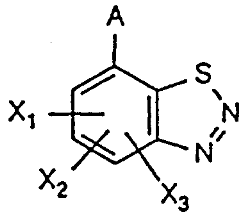

(I)

dans laquelle :

$X_1$, $X_2$ et $X_3$ sont un atome d'hydrogène ;
A représente un groupe alkyle en $C_1$ à $C_2$, substitué par au plus 3 groupes X-alkyle en $C_1$ à $C_4$, un groupe méthyle substitué par 2 ou 3 atomes d'halogène, un groupe éthyle substitué par un hydroxy et/ou par jusqu'à 4 atomes d'halogène inclus, un groupe vinyle non substitué ou portant jusqu'à 3 atomes d'halogène inclus, ou encore un groupe éthinyle, propargyle, formyle, acétyle, acétyle substitué par au plus 3 atomes d'halogène ou l'un des groupes suivants $C(R)=N-N(R_2)R_3$, $C(N=N-U_1)=N-NH-U_1$, $CH(R)-[N(R_1)]_n-N(R_2)R_3$, $C(R)(CN)OR_4$, $C(R)=N(O)_nR_3$, $CH(R)-O-N=C(R_1)R_2$, $CH(R)-O-N=C(CN)-CONH-R_5$, $C(R_6)=N-(O)_nR$, $CH(R)-Y-E-R_3$, $CO-[C(OR)_2]_nQ$, $C(Q)=CH-OR$ ou T-Q ;
dans laquelle en outre :
n vaut 0 ou 1;
X et Y, indépendamment l'un de l'autre, sont un atome d'oxygène ou de soufre ;
R et $R_1$, indépendamment l'un de l'autre, sont un atome d'hydrogène ou un groupe alkyle en $C_1$ à $C_2$;
$R_2$ est un atome d'hydrogène, un groupe alkyle en $C_1$ à $C_8$, un groupe alcényle en $C_3$ à $C_6$, un groupe alcinyle en $C_3$ à $C_6$, un groupe cycloalkyle en $C_3$ à $C_7$, un groupe benzyle ou cyano ;
$R_3$ est un atome d'hydrogène, un groupe alkyle en $C_1$ à $C_6$, un groupe alcényle en $C_3$ à $C_6$, un groupe alcinyle en $C_3$ à $C_6$, un groupe cycloalkyle en $C_3$ à $C_7$, un groupe benzyle ou un reste aryle U ;
$R_4$ est un atome d'hydrogène, un groupe alkyle en $C_1$ à $C_6$, un groupe Si(alkyle en $C_1$ à $C_6)_3$ ou un groupe OCO -alkyle en $C_1$ à $C_3$ ;
$R_5$ est un atome d'hydrogène ou $CONHR_1$ ;
$R_6$ est $N(R_1)R_2$, un hydrazino ou Q ;
E est CO ou $SO_2$ ;
U et $U_1$, indépendamment l'un de l'autre, sont un reste phényle non substitué ou mono ou polysubstitué, de façon identique ou différente, par un méthyle, un méthoxy, un halogène, un trifluorométhyle, un nitro ou un cyano;
T est un alkylène en $C_1$ à $C_2$, indépendamment les uns des autres un méthylène substitué par un amino, un hydroxy ou un halogène, ou un éthénylène non substitué ou substitué par un halogène ou un cyano ;
Q est COXR ou un groupe cyano.

**2.** Utilisation selon la revendication 1, caractérisée en ce que, dans le composé de formule I :

$X_1$, $X_2$ et $X_3$ sont un atome d'hydrogène ;
A représente un groupe alkyle en $C_1$ à $C_2$, substitué par au plus 3 groupes X-alkyle en $C_1$ à $C_4$, un groupe méthyle substitué par 2 ou 3 atomes d'halogène, un groupe éthyle substitué par un hydroxy et/ou par jusqu'à 4 atomes d'halogène inclus, ou encore un groupe formyle, un groupe acétyle ou l'un des groupes suivants $C(R)=N-N(R_2)R_3$, $CH(R)-[N(R_1)]_n-N(R_2)R_3$, $C(R)(CN)OR_4$, $C(R)=N(O)_nR_3$, $CH(R)-O-N=C(R_1)R_2$, CH(R)-O-

$N=C(CN)-CONH-R_5$, $C(R_6)=N-(O)_nR$, $CH(R)-Y-E-R_3$, $CO-[C(OR)_2]_nCOXR$, $C(Q)=CH-OR$ ou T-Q ;
dans laquelle en outre :

n vaut 0 ou 1 ;
X et Y sont un atome d'oxygène ;
R et $R_1$, indépendamment l'un de l'autre, sont un atome d'hydrogène ou un groupe alkyle en $C_1$ à $C_2$ ;
$R_2$ est un atome d'hydrogène, un groupe alkyle en $C_1$ à $C_4$, un groupe alcényle en $C_3$ à $C_4$, un groupe alcinyle en $C_3$ à $C_4$, un groupe cycloalkyle en $C_3$ à $C_6$, ou un groupe benzyle ;
$R_3$ est un atome d'hydrogène, un groupe alkyle en $C_1$ à $C_4$, un groupe alcényle en $C_3$ à $C_4$, un groupe alcinyle en $C_3$ à $C_4$, un groupe cycloalkyle en $C_3$ à $C_6$, ou un reste phényle substitué, de façon identique ou différente, par un méthyle, un halogène ou un trifluorométhyle;
$R_4$ est un atome d'hydrogène, un groupe alkyle en $C_1$ à $C_3$ ou un groupe Si(alkyle en $C_1$ à $C_2)_3$ ;
$R_5$ est un atome d'hydrogène ou CONH-alkyle en $C_1$ à $C_3$ ;
$R_6$ est un groupe amino ou cyano ;
E est CO ;
T est un groupe méthylène, un groupe méthylène substitué par un amino ou un groupe éthénylène ;
Q est COOR ou un groupe cyano.

**3.** Utilisation selon la revendication 1, caractérisée en ce que, dans le composé de formule I :

$X_1$, $X_2$ et $X_3$ sont un atome d'hydrogène ;
A représente un groupe méthyle substitué par au plus 2 groupes X-alkyle en $C_1$ à $C_4$, un groupe méthyle substitué par 2 ou 3 atomes de fluor ou de chlore, un groupe éthyle substitué par un hydroxy ou un chlore, ou un groupe formyle, un groupe acétyle ou encore l'un des groupes suivants $C(R)=N-N(R_2)R_3$, $CH(R)-[N(R_1)]_n-N(R_2)R_3$, $C(R)(CN)OR_4$, $C(R)=N(O)_nR_3$, $CH(R)-O-N=C(R_1)R_2$, $CH(R)-O-N=C(CN)-CONHR_5$, $C(R_6)=N-OR$ $CH(R)-Y-E-R_3$, $CO-[C(OR)_2]_nCOXR$, $C(COOR)=CH-OR$ ou T-Q ;
dans laquelle en outre :
n vaut 1 ;
X est un atome d'oxygène ;
R et $R_1$, indépendamment l'un de l'autre, sont un atome d'hydrogène ou un groupe méthyle ;
$R_2$ est un atome d'hydrogène, un groupe alkyle en $C_1$ à $C_2$, un groupe alkyle, propargyle, cyclopropyle ou benzyle ;
$R_3$ est un atome d'hydrogène, un groupe alkyle en $C_1$ à $C_2$, un groupe allyle, propargyle, cyclopropyle ou un reste phényle substitué, de façon identique ou différente, par un méthyle, un fluor, un chlore ou un trifluorométhyle;
$R_4$ est un atome d'hydrogène, un groupe alkyle en $C_1$ à $C_2$ ou $Si(CH_3)_3$ ;
$R_5$ est un atome d'hydrogène ou CONH-alkyle en $C_1$ à $C_2$ ;
$R_6$ est un groupe amino ;
Y est un atome d'oxygène ;
E est CO ;
T est un groupe méthylène ou un groupe cyano.

**4.** Utilisation selon la revendication 1, caracterisée en ce que, dans le composé de formule I :

$X_1$, $X_2$ et $X_3$ sont un atome d'hydrogène ;
A représente un groupe méthyle substitué par au plus 2 groupes X-alkyle en $C_1$ à $C_2$, un groupe méthyle substitué par 2 ou 3 atomes de fluor, un groupe éthyle substitué par un hydroxy ou un chlore, ou un groupe formyle, ou encore l'un des groupes suivants $C(R)=N-N(R_2)R_3$, $CH(R)-[N(R_1)]_n-N(R_2)R_3$, $C(R)(CN)OR_4$, $C(R)=N-R_3$, $CH(R)-O-N=C(R_1)R_2$, $C(R_6)=N-OR$, $CH(R)-Z-E-R_3$, $CO-[C(OR)_2]_nCOXR$, ou T-Q ;
dans laquelle en outre :
n vaut 1 ;
X est un atome d'oxygène ;
R et $R_1$, indépendamment l'un de l'autre, sont un atome d'hydrogène ou un groupe méthyle ;
$R_2$ est un atome d'hydrogène, un groupe méthyle, un groupe allyle, un groupe cyclopropyle ou un groupe benzyle ;
$R_3$ est un atome d'hydrogène, un groupe méthyle, un groupe allyle, un groupe cyclopropyle ou un reste phényle substitué, de façon identique ou différente, par un méthyle, un fluor, un chlore ou un trifluorométhyle ;
$R_4$ est un atome d'hydrogène un groupe méthyle ou $Si(CH_3)_3$ ;
$R_5$ est un atome d'hydrogène ou $CONH-CH_2CH_3$ ;

$R_6$ est un groupe amino ;
Y est un atome d'oxygène ;
E est CO ;
T est un groupe méthylène ;
Q est $COOCH_3$ ou un groupe cyano.

**5.** Utilisation selon la revendication 1, caractérisée en ce que le composé est sélectionné dans le groupe composé de :

7-formyl-1,2,3-benzothiadiazole ;
7-acétoxyméthyl-1,2,3-benzothiadiazole ;
7-[méthoxyimino-(2-cyanoacétamyl)]-1,2,3-benzothiadiazole ;
7-(N-méthoxyimino-méthyl)-1,2,3-benzothiadiazole ;
7-(N-méthoxyimino-hydroxyméthyl)-1,2,3-benzothiadiazole ;
7-méthoxyméthyl-1,2,3-benzothiadiazole ;
acide 3-(7-benzo-1,2,3-thiadiazole)-acrylique ;
7-cyanométhyl-1,2,3-benzothiadiazole ;
7-trichlorométhyl-1,2,3-benzothiadiazole ;
7-dichlorométhyl-1,2,3-benzothiadiazole ;
benzo-1,2,3-thiadiazol-7-(N-hydroxycarboximido-amide) ;
benzo-1,2,3-thiadiazol-7-(acide N-méthoxyhydroxamique) ;
2-(benzo-1,2,3-thiadiazolyl)-2-hydroxyiminoacétonitrile ;
5-fluoro-benzo-1,2,3-thiadiazol-7-carbaldéhyde ;
6-fluoro-benzo-1,2,3-thiadiazol-7-carbaldéhyde ;
4-fluoro-benzo-1,2,3-thiadiazol-7-carbaldéhyde ;
N,N-diphényl-C-[benzo-1,2,3-thiadiazol-7'yl]formazan ;
7-acétyl-benzo-1,2,3-thiadiazole ;
7-(bromoacétyl)-benzo-1,2,3-thiadiazole.

**6.** Composés de formule I selon la revendication 1, exception faite des composés suivants :

7-formyl-1,2,3-benzothiadiazole ;
7-acétyl-1,2,3-benzothiadiazole ;
6-chloro-7-formyl-1,2,3-benzothiadiazole ;
6-méthylthio-7-formyl-1,2,3-benzothiadiazole ;
4-bromo-6-chloro-7-formyl-1,2,3-benzothiadiazole ;
6-méthoxy-7-formyl-1,2,3-benzothiadiazole ;
7-hydroxyimino-1,2,3-benzothiadiazole ;
6-méthoxy-7-oximino-1,2,3-benzothiadiazole ;
7-dibromoacétyl-1,2,3-benzothiadiazole.

**7.** Composés de formule I selon la revendication 2, exception faite des composés suivants :

7-formyl-1,2,3-benzothiadiazole ;
7-acétyl-1,2,3-benzothiadiazole ;
6-chloro-7-formyl-1,2,3-benzothiadiazole ;
6-méthylthio-7-formyl-1,2,3-benzothiadiazole ;
4-bromo-6-chloro-7-formyl-1,2,3-benzothiadiazole ;
6-méthoxy-7-formyl-1,2,3-benzothiadiazole ;
7-hydroxyimino-1,2,3-benzothiadiazole ;
6-méthoxy-7-oximino-1,2,3-benzothiadiazole ;
7-dibromoacétyl-1,2,3-benothiadiazole.

**8.** Composés de formule I selon la revendication 3, exception faite des composés suivants :

7-formyl-1,2,3-benzothiadiazole ;
7-acétyl-1,2,3-benzothiadiazole ;
6-chloro-7-formyl-1,2,3-benzothiadiazole ;

6-méthylthio-7-formyl-1,2,3-benzothiadiazole ;
4-bromo-6-chloro-7-formyl-1,2,3-benzothiadiazole ;
6-méthoxy-7-formyl-1,2,3-benzothiadiazole ;
7-hydroxyimino-1,2,3-benzothiadiazole ;
6-méthoxy-7-oximino-1,2,3-benzothiadiazole ;
7-dibromoacétyl-1,2,3-benzothiadiazole.

**9.** Composés de formule I selon la revendication 4, exception faite des composés suivants :

7-formyl-1,2,3-benzothiadiazole ;
7-acétyl-1,2,3-benzothiadiazole ;
6-chloro-7-formyl-1,2,3-benzothiadiazole ;
6-méthylthio-7-formyl-1,2,3-benzothiadiazole ;
4-bromo-6-chloro-7-formyl-1,2,3-benzothiadiazole ;
6-méthoxy-7-formyl-1,2,3-benzothiadiazole ;
7-hydroxyimino-1,2,3-benzothiadiazole ;
6-méthoxy-7-oximino-1,2,3-benzothiadiazole ;
7-dibromoacétyl-1,2,3-benzothiadiazole.

**10.** Composé de formule I sélectionné dans le groupe composé de :

7-acétoxyméthyl-1,2,3-benzothiadiazole ;
7-[méthoxyimino-(2-cyanoacétamyl)]-1,2,3-benzothiadiazole ;
7-(N-méthoxyimino-méthyl)-1,2,3-benzothiadiazole ;
7-(N-méthoxyimino-hydroxyméthyl)-1,2,3-benzothiadiazole ;
7-méthoxyméthyl-1,2,3-benzothiadiazole ;
acide 3-(7-benzo-1,2,3-thiadiazol)-acrylique 7-cyanométhyl-1,2,3-benzothiadiazole ;
7-trichlorométhyl-1,2,3-benzothiadiazole ;
7-dichlorométhyl-1,2,3-benzothiadiazole ;
benzo-1,2,3-thiadiazol-7-(N-hydroxycarboximido-amide) ;
benzo-1,2,3-thiadiazol-7-(acide N-méthoxyhydroxamique) ;
2-(benzo-1,2,3-thiadiazolyl)-2-hydroxyimino-acétonitrile ;
5-fluoro-benzo-1,2,3-thiadiazol-7-carbaldéhyde ;
6-fluoro-benzo-1,2,3-thiadiazol-7-carbaldéhyde ;
4-fluoro-benzo-1,2,3-thiadiazol-7-carbaldéhyde ;
N,N-diphényl-C-[benzo-1,2,3-thiadiazol-7'yl]formazan ;
7-(bromoacétyl)-benzo-1,2,3-thiadiazole.

**11.** Utilisation selon la revendication 1, caractérisée en ce que des plantes utiles sont immunisées contre des maladies.